# EUROPÄISCHE PATENTSCHRIFT

(11) **EP 4 285 974 B1**
(45) Veröffentlichungstag und Bekanntmachung des Hinweises auf die Patenterteilung: **17.09.2025**
(21) Anmeldenummer: 23171467.6
(22) Anmeldetag: 04.05.2023
(51) Int. Cl.: A61M 16/00

(54) **BEATMUNGSGERÄTES**
VENTILATOR
APPAREIL RESPIRATOIRE

(30) Priorität: 01.06.2022 DE 102022113877
(43) Veröffentlichungstag der Anmeldung: 06.12.2023
(73) Patentinhaber: Löwenstein Medical Technology S.A., 2557 Luxembourg (LU)
(72) Erfinder: Schattner, Jan, 25421 Pinneberg (DE); Verhoeven, Jan, 76275 Ettlingen (DE); Göbel, Christof, 22457 Hamburg (DE)
(74) Vertreter: Löwenstein Medical IP

(56) Entgegenhaltungen:
- EP-A1- 3 501 583
- WO-A1-2014/076618
- US-A1- 2007 186 928
- US-A1- 2007 199 566
- US-A1- 2017 007 494
- US-A1- 2018 043 116

## Beschreibung

Die Offenbarung betrifft ein System und ein Therapiegerät mit Mechanismen zum automatischen Start einer Therapie und/oder Sekretmanagement oder die automatische Erinnerung daran.

Im Stand der Technik sind mechanische Insufflatoren/Exsufflatoren bekannt, um Patienten bei dem Abhusten von Sekret zu unterstützen. Die mechanischen Insufflatoren/Exsufflatoren werden in der Regel unabhängig von Beatmungsgeräten genutzt. Zur Therapie muss dazu vom Beatmungsgerät auf das Therapiegerät gewechselt werden. Da die mechanischen Insufflatoren/Exsufflatoren selten genutzt werden und die Nutzung zum Teil keine besonders angenehme Erfahrung ist, kann der Patient eine regelmäßige Nutzung schnell vergessen. Dabei können sich größere Mengen an Sekret in den Atemwegen des Patienten ansammeln, was zu einer Verschlechterung der Beatmungssituation führen kann. Eine prophylaktische Nutzung eines mechanischen In-/Exsufflators oder eine prophylaktische (manuelle) Absaugung von Sekret verbessert dabei die Beatmungssituation und führen dazu, dass regelmäßig die Lungen von Sekret befreit werden. Neben den Insufflatoren/Exsufflatoren sind auch Absauggeräte bekannt, über welche je nach verfügbarer Zeit zum Beispiel im Krankhaus eingesetzt werden, um etwa Sekret aus den oberen Atemwegen oder dem Mundbereich zu entfernen.

EP 3 501 583 A1 beschreibt ein Atemtherapiesystem mit zwei separaten und unabhängig voneinander betreibbaren Atemtherapiegeräten, beispielsweise einem Beatmungsgerät und einem Hustengerät. Über das Beatmungsgerät kann eine Benachrichtigung ausgegeben werden, die auf eine erneute Durchführung der therapeutischen Atemunterstützung mittels des Hustengeräts hinweist. Das Beatmungsgerät kann ausgebildet sein, um Parameter wie Fluss, Druck, Sauerstoffsättigung, Frequenz oder Volumen während der Beatmung zu erfassen und auszuwerten, um eine mögliche Verschlechterung der Patientenatmung zu identifizieren und daraufhin die Benachrichtigung zu generieren.

Aufgabe der vorliegenden Offenbarung ist ein System für ein regelmäßiges und bedarfsgerechtes Sekretmanagement bereitzustellen.

Die Erfindung ist in den angehängten Ansprüchen definiert. Die Offenbarung betrifft ein System mit zumindest einem Therapiegerät zur Durchführung einer Therapie, und mindestens ein Schlauchsystem zur gasleitenden Verbindung zwischen dem Therapiegerät und einem Patienten, wobei das Therapiegerät zumindest eine Quelle für Gas zur Erzeugung eines Überdrucks und/oder zumindest eine Senke für Gas zur Erzeugung eines Unterdrucks aufweist und zumindest zeitweise dem Schlauchsystem mit einen Überdruck und/oder Unterdruck zur Durchführung der Therapie bereitzustellen, und wobei das System ferner zumindest eine Steuereinheit, zumindest ein Benutzerschnittstelle, zumindest einen Speicher und zumindest einen Sensor umfasst. Das System kennzeichnet, dass die Steuereinheit einen Therapiestartmechanismus zum Starten der Therapie, und/oder einen Therapieerinnerungsmechanismus zur Erinnerung an die Therapie und/oder einen Therapiewiederholungsmechanismus zur Verlängerung und/oder Wiederholung der Therapie aufweist, die jeweils auf Nutzeraktionen, Patienten- und/oder Therapiedaten basieren.

In manchen Ausführungsformen kennzeichnet das System, dass die Steuereinheit eingerichtet ist, über das Benutzerschnittstelle und/oder eine externe Schnittstelle die Erinnerung an die Therapie und/oder den Start der Therapie und/oder die Wiederholung und/oder die Verlängerung der Therapie einstellbar zu machen.

In manchen Ausführungsformen kennzeichnet das System, dass der Therapiestartmechanismus und/oder der Erinnerungsmechanismus und/oder der Therapiewiederholungsmechanismus über die Benutzerschnittstelle abbrechbar und/oder zeitlich verschiebbar sind.

In manchen Ausführungsformen kennzeichnet das System, dass die Steuereinheit eingerichtet ist, über das Benutzerschnittstelle und/oder über die Schnittstelle und/oder aus dem Speicher den Patienten charakterisierende Daten, bspw. zur Lungenfunktion (Vitalkapazität/Insufflationskapazität, Lungen-/Thoraxcompliance etc.), Alter, Gewicht, Beatmungsdaten, Daten zur Pathologie/Grunderkrankung, Vorerfahrungen mit HustenManövern, für eine Häufigkeit der Erinnerungen und/oder Starts der Therapie automatisch anzupassen.

In manchen Ausführungsformen kennzeichnet das System, dass auf Basis von ermittelten Daten, insbesondere basierend auf Messwerten und gespeicherten Werten zu SpO2, Puls, und/oder CO2 und/oder sich veränderten Druck-/ und Volumenflussverhalten, die Therapie automatisch gestartet oder dem Patienten der Start der Therapie angeboten wird und/oder an die Therapie erinnert wird, wenn diese Daten anzeigen, dass eine Sekretentfernung aus den Atemwegen durch eine Therapie förderlich wäre.

In manchen Ausführungsformen kennzeichnet das System, dass das Therapiegerät mit einem Beatmungsgerät gekoppelt ist oder das Therapiegerät und das Beatmungsgerät als ein Gerät kombiniert sind und die Daten vom Beatmungsgerät bereitgestellt werden.

Falls das Therapiegerät nur Überdruck erzeugt, ist es sinnvoll, wenn das Therapiegerät und das Beatmungsgerät als ein und dasselbe Gerät ausgeführt sind. Beispielsweise kann das Therapiegerät als mindestens ein Hard- und/oder Softwaremodul des Beatmungsgeräts ausgeführt sein.

In manchen Ausführungsformen kennzeichnet das System, dass die zugrundeliegenden Daten insbesondere den SpO2 Wert umfassen, wobei bei Erreichen eines Schwellwertes der SpO2 Sättigung ein Therapiestart und/oder eine Therapieerinnerung und/oder eine Therapiewiederholung und/oder eine Therapieverlängerung erfolgt.

In manchen Ausführungsformen kennzeichnet das System, dass der SpO2 Schwellwert in dem System hinterlegt ist und optional einstellbar ist.

In manchen Ausführungsformen kennzeichnet das System, dass die Wirkung der durchgeführten Therapie auf den Verlauf von mindestens einer der Größen SpO2, CO2, Druck, Fluss, Tidalvolumen, akustische Signale/Töne Signal-Rausch-Verhältnis (SNR) im Therapiegerät und/oder in einem gekoppelten Beatmungsgerät gespeichert und/oder verarbeitet wird und wobei diese gespeicherten und/oder verarbeiteten Daten bei Entscheidungen zum Therapiestart und/oder Therapiewiederholungen und/oder Therapieerinnerungen berücksichtigt werden.

In manchen Ausführungsformen kennzeichnet das System, dass das System dazu eingerichtet ist, die zugrundeliegenden Daten, insbesondere das Signal-Rausch-Verhältnis (SNR) der Druck- und/oder Flusssignale, zu evaluieren und bei Erreichen einer Schwelle des SNR Werts den Therapiestart oder eine Erinnerung daran einleitet oder verlängert.

In manchen Ausführungsformen kennzeichnet das System, dass die Erinnerung oder die Möglichkeit zum Starten des Manövers ausschließlich und/oder zusätzlich über das gekoppelte Beatmungsgerät erfolgt.

In manchen Ausführungsformen kennzeichnet das System, dass der Therapiestart und/oder die Therapieerinnerung und/oder die Therapiewiederholung bestimmte Parameter auf dem Beatmungsgerät verändern.

In manchen Ausführungsformen kennzeichnet das System, dass eine ermittelte Auswirkung von durchgeführten Therapien auf den Verlauf von mindestens einer der Größen SpO2, CO2, Fluss, Tidalvolumen, Signal-Rausch-Verhältnis (SNR) die Beatmungsparameter auf dem Beatmungsgerät verändern.

In manchen Ausführungsformen kennzeichnet das System, dass das Therapiegerät und das Beatmungsgerät pneumatisch koppelbar sind, sodass eine Beatmung und eine maschinelle Insufflation und Exsufflation direkt kombiniert werden.

In manchen Ausführungsformen kennzeichnet das System, dass die beiden Gerätefunktionen Beatmung und maschinelle In/-Exsufflation in einem Gerät realisiert sind.

In manchen Ausführungsformen kennzeichnet das System, dass auf Basis der ermittelten Daten, insbesondere basierend auf Messwerten und gespeicherten Werten zu Druck, Fluss, Tidalvolumen, SpO2, Puls, und/oder CO2 oder Daten aus vergangenen Hustenmanövern, wie bspw. dem Peak Cough Flow (PCF) oder der Zeitpunkte der Therapiestarts- und/oder Erinnerungen und/oder Therapiewiederholungen, Wiederholungen von In- und Exsufflationen automatisch durchgeführt und/oder dem Patienten angeboten werden, wenn diese Daten anzeigen, dass weiteres Sekret aus den Atemwegen entfernt sollte.

In manchen Ausführungsformen kennzeichnet das System, dass ermittelte und/oder gespeicherte Daten in ein cloudbasiertes System hochgeladen werden und dass cloudbasierte Algorithmen therapiebezogene Entscheidungen treffen und dass diese Entscheidungen dem Therapiegerät wiederum aus der Cloud für die Anpassung der Therapie oder von Startzeitpunkten der Therapie zur Verfügung gestellt werden.

In manchen Ausführungsformen kennzeichnet das System, dass anwenderseitiges Feedback zur subjektiven Wahrnehmung von automatisch durchgeführten Therapiestarts und/oder Wiederholungen und/oder Erinnerungen an der Nutzerschnittstelle und/oder über eine App auf einem mit dem Therapiegerät gekoppelten mobilen Endgerät möglich sind und wobei dieses anwenderseitige Feedback gespeichert und/oder verarbeitet wird und bei folgenden Entscheidungen zum Therapiestart und/oder Therapiewiederholungen und/oder Erinnerungen an einen Therapiestart berücksichtigt wird.

In manchen Ausführungsformen kennzeichnet das System, dass die Hustentherapie in Folge einer Therapieerinnerung durch das Therapiegerät über eine über eine Fernsteuerung und/oder eine App auf einem mit dem Therapiegerät gekoppelten mobilen Endgerät anwenderseitig gestartet und/oder wiederholt und/oder beendet werden kann.

In manchen Ausführungsformen kennzeichnet das System, dass auf Basis von ermittelten Daten ein automatischer Therapiestart vorgesehen ist, wobei die Therapieparameter anhand der ermittelten Daten automatisch angepasst wird.

In manchen Ausführungsformen kennzeichnet das System, dass auf dem Therapiegerät und/oder einem gekoppelten Beatmungsgerät eine Meldung generiert wird, welche auf den automatischen Therapiestart hinweist.

In manchen Ausführungsformen kennzeichnet das System, dass der automatische Therapiestart über die Benutzerschnittstelle abbrechbar und/oder zeitlich verschiebbar ist.

In manchen Ausführungsformen kennzeichnet das System, dass das Therapiegerät zumindest eine Quelle für Gas zur Erzeugung eines Überdrucks umfasst, um zur Durchführung der Therapie dem Schlauchsystem zumindest zeitweise Überdruck für eine Insufflation bereitzustellen.

In manchen Ausführungsformen kennzeichnet das System, dass das Therapiegerät zumindest eine Senke für Gas zur Erzeugung eines Unterdrucks umfasst, um zur Durchführung der Therapie dem Schlauchsystem zumindest zeitweise Unterdruck zum Absaugen von Sekret und/oder für eine Exsufflation bereitzustellen.

In manchen Ausführungsformen kennzeichnet das System, dass das Therapiegerät zumindest eine Quelle für Gas zur Erzeugung eines Überdrucks und mindestens eine Senke für Gas zur Erzeugung eines Unterdrucks sowie ein Schaltmittel umfasst, wobei das Schaltmittel dazu eingerichtet ist dem Schlauchsystem zumindest zeitweise Überdruck für eine Insufflation und/oder Unterdruck für eine Exsufflation bereitzustellen wobei die Steuereinheit eingerichtet ist
an dem Schlauchsystem Überdruck unter einem vorbestimmten Druck und/oder Druckverlauf und für eine vorbestimmte Zeitdauer zur Insufflation bereitzustellen, und vorzugsweise nachfolgend Unterdruck unter einem vorbestimmten Druck und/oder Druckverlauf und für eine vorbestimmte Zeitdauer zur Exsufflation bereitzustellen.

In manchen Ausführungsformen kennzeichnet das System, dass zumindest eine Steuereinheit, zumindest ein Speicher, zumindest eine Benutzerschnittstelle und zumindest ein Sensor Teil des Therapiegerätes sind.

Die Offenbarung betrifft ferner ein Therapiegerät, umfassend zumindest eine Quelle für Gas zur Erzeugung eines Überdrucks, wobei das Therapiegerät ferner zumindest eine Steuereinheit, ein Benutzerschnittstelle, einen Speicher und einen Sensor umfasst, wobei das Therapiegerät mit zumindest einem Schlauchsystem zur Verbindung eines Patienten mit dem Therapiegerät verbindbar ist und das Therapiegerät mindestens ein Schaltmittel aufweist, welches dazu eingerichtet ist an dem Schlauchsystem zumindest zeitweise einen Überdruck zur Insufflation bereitzustellen. Das Therapiegerät kennzeichnet, dass die Steuereinheit einen Therapiestartmechanismus zum Starten der Therapie und/oder Erinnerungsmechanismus zur Erinnerung an die Therapie und/oder einem Therapiewiederholungsmechanismus zur Verlängerung und/oder Wiederholung der Therapie aufweist, die jeweils auf Nutzeraktionen, Patienten- und/oder Therapiedaten basieren.

In manchen Ausführungsformen kennzeichnet das, dass das Therapiegerät ferner eine Senke für Gas zur Erzeugung eines Unterdrucks umfasst wobei das Schaltmittel dazu eingerichtet ist dem Schlauchsystem zumindest zeitweise Überdruck für eine Insufflation und/oder Unterdruck für eine Exsufflation bereitzustellen und die Steuereinheit dazu eingerichtet ist,
a. an dem Schlauchsystem Überdruck unter einem vorbestimmten Druck und/oder Druckverlauf und für eine vorbestimmte Zeitdauer zur Insufflation bereitzustellen, und
b. vorzugsweise nachfolgend Unterdruck unter einem vorbestimmten Druck und/oder Druckverlauf und für eine vorbestimmte Zeitdauer zur Exsufflation bereitzustellen.

In manchen Ausführungsformen kennzeichnet das, dass das Therapiegerät auch eine Funktion zur Beatmung umfasst und/oder mit einem Beatmungsgerät koppelbar ist.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des offenbarungsgemäß Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen zumindest ausatmet und/oder einatmet.

Im Sinne der Offenbarung ist eine Quelle für Gas zur Erzeugung eines Überdrucks gleichbedeutend mit einer Quelle für Überdruckgas. Eine Senke für Gas zur Erzeugung eines Unterdrucks ist gleichbedeutend mit einer Quelle für Unterdruckgas. Es wird ausdrücklich darauf hingewiesen, dass eine Quelle für Überdruckgas auch gleichzeitig eine Quelle für Unterdruckgas darstellen kann. Je nach Ausführungsform ist also eine gemeinsame Quelle für Überdruckgas und Unterdruckgas zu finden. Alternativ oder ergänzend können aber auch separate Quellen für Überdruckgas und Unterdruckgas angeordnet sein.

Im Zuge der Beschreibung der Offenbarung bezeichnet der Begriff "Therapie" soweit nicht anders beschrieben allgemein ein Sekretmanagement. Das Sekretmanagement kann beispielsweise eine Hustentherapie, eine Hustenunterstützung, eine Insufflation, eine Insufflation mit gefolgter Exsufflation und/oder auch das Absaugen von Sekret aus dem Mund oder den Atemwegen umfassen. Auch das Absaugen von Sekret über eine Kanüle, etwa bei Patienten, welche über eine Trachealkanüle beatmet werden, kann im Sinne der Offenbarung als Therapie angesehen werden. Durch eine Insufflation, optional mit anschließendem Exsufflation, wird der behandelte Patient beispielsweise dazu bewegt und /oder dabei unterstützt zu husten. Das Husten kann dabei zum Beispiel dienen Sekret aus den Atemwegen und/oder der Lunge abzuhusten bzw. zu entfernen. Eine Therapie umfasst dabei in der Regel mehrere Hustenmanöver, welche wiederum in mehrere Hustenzyklen unterteilt sind. Ein Hustenzyklus umfasst dabei in der Regel zumindest eine Insufflation und eine Exspiration bzw. Husten. In manchen Ausführungsformen umfasst ein Hustenzyklus zumindest eine Insufflation und eine Exsufflation.

Eine Insufflation beschreibt ein druckunterstütze vertiefte Inspiration des Patienten, wobei der Inspirationsdruck und ggf. die Inspirationslänge größer als bei einer normalen Inspiration im Rahmen einer (maschinellen) Beatmung sind. Damit zusammenhängend können auch Fluss- und/oder Volumina größer sein. Die Insufflation kann beispielsweise auch über mehrere Stufen durchgeführt werden, zwischen denen anstelle der Exspiration eine Pause liegt (Air Stacking). Die Insufflation kann alternativ in einem einzelnen Hub durchgeführt werden. Der einzelne Hub kann beispielhaft gegenüber der normalen Inspiration verlängert und/oder vertieft sein. Nach der Insufflation folgt beispielsweise eine Exsufflation oder der Patient kann eine tiefe und forcierte Exspiration (Husten) durchführen.

Eine Exsufflation beschreibt ein, optional plötzliches, Bereitstellen von Unterdruck (bezogen auf den normalen Umgebungsdruck) nach der Insufflation, wodurch eine tiefe Exspiration bzw. ein Husten des Patienten forciert wird. Die Bereitstellung eines Unterdrucks führt dabei zu einer höheren Druckdifferenz und kann ein effizienteres Husten ermöglichen.

Der Therapiestart kann durch verschiedene Aspekte gekennzeichnet sein, welche zum Teil von den jeweiligen Vorrausetzungen abhängen. Ist das Therapiegerät ein eigenständiges Gerät, sodass Patienteninterface und/oder Schlauch gewechselt werden muss, kann ein Therapiestart beispielsweise beim Wechsel des Patienteninterface angesehen werden. Ein Therapiestart kann auch mit dem Einschalten und/oder Vorbereiten des Therapiegerätes definiert sein. In manchen Ausführungsformen ist der Therapiestart der Zeitpunkt, ab welchem das Therapiegerät das erste Hustenmanöver startet und/oder vorbereitet. Eine Vorbereitung können etwa ein oder mehrere Atemzüge vor dem ersten Hustenzyklus sein. Auch das Aufwärmen und/oder Hochfahren des Therapiegerätes und/oder Aktivierung des Gebläses kann als Therapiestart angesehen werden.

Das Therapieende kann mit Beendigung des letzten Therapiezyklus definiert sein. Auch kann als Therapieende angesehen werden, wenn das Therapiegerät ausgeschaltet bzw. deaktiviert wird. Ist das Therapiegerät beispielsweise mit einem Beatmungsgerät zumindest für eine Datenübertragung verbunden, so ist eine Deaktivierung des Therapiegerätes der Zustand, wenn das Therapiegerät nicht mehr aktiv eine Therapie durchführt und/oder nur noch Daten mit dem gekoppelten Beatmungsgerät austauscht. Auch ein Wechsel bzw. Entfernen des Patienteninterface für die Hustentherapie kann als Therapieende gelten.

Das System umfasst zumindest einen Mechanismus zur Erinnerung an eine Therapie und/oder einen Mechanismus zum Starten einer Therapie und/oder einen Mechanismus zum Wiederholen und/oder Verlängern einer Therapie. Es kann vorgesehen sein, dass das System zumindest zwei oder alle drei der Mechanismen umfasst. Die Mechanismen können dabei optional aktiviert und/oder deaktiviert werden. Es kann dabei auch vorgesehen sein, dass zumindest einer der Mechanismen automatisch aktiviert und/oder deaktiviert wird. Wird beispielsweise ein Therapiegerät mit einem Beatmungsgerät gekoppelt, kann zumindest einer der Mechanismen aktiviert werden. Hierbei bedeutet eine Aktivierung/Deaktivierung nicht, dass unmittelbar eine Therapie durchgeführt wird, sondern, dass der nun aktivierte Mechanismus entscheiden kann, wann eine Therapie durchgeführt werden soll und diese gegebenenfalls startet oder einen entsprechenden Hinweis generiert.

Das System umfasst dabei zumindest eine Steuereinheit, zumindest einen Speicher, zumindest eine Benutzerschnittstelle und zumindest einen Sensor. Die Steuereinheit ist dabei so eingerichtet und ausgebildet, dass sie den Therapiestartmechanismus und/oder den Therapiewiederholungsmechanismus und/oder den Therapieerinnerungsmechanismus aufweist. In manchen Ausführungsformen ist die Steuereinheit Teil des Therapiegerätes. Es kann aber auch vorgesehen sein, dass die Steuereinheit zumindest örtlich unabhängig vom Therapiegerät im System angeordnet ist. Beispielsweise kann die Steuereinheit in einem Beatmungsgerät angeordnet sein, welches optional mit dem Therapiegerät gekoppelt ist.

Das System umfasst zumindest ein Therapiegerät, welches zur Durchführung der Therapie ausgebildet ist.

In manchen Ausführungsformen umfasst das System zumindest ein Therapiegerät, welches dazu ausgebildet ist mittels zumindest einer Quelle für Gas einen Überdruck zu erzeugen und ein Schlauchsystem mit diesem Überdruck zu beaufschlagen. Das Therapiegerät ist beispielhaft dazu eingerichtet, mit dem Überdruck eine Insufflation durchzuführen. Das System umfasst beispielhaft zusätzlich ein Schaltmittel, welches durch ein plötzliches Umschalten die Beaufschlagung des Schlauchsystems mit Überdruck stoppt, wodurch bei Patienten ein plötzlicher Druckabfall entsteht und den Patienten gegebenenfalls zu einer tiefen, forcierten Exspiration bzw. einem Husten bringt.

In manchen Ausführungsformen umfasst das Therapiegerät alternativ oder ergänzend eine Senke für Gas zur Erzeugung eines Unterdrucks. Über den Unterdruck kann in manchen Ausführungsformen Sekret aus den Atemwegen eines Patienten abgesaugt werden. Die Senke kann in machen Ausführungsformen auch mit einer Quelle für Gas zur Erzeugung eines Überdrucks kombiniert sein, sodass die Senke gleichzeitig bzw. zeitweise auch als Quelle für Gas dienen kann. Beispielsweise kann ein Therapiegerät gleichzeitig bzw. zeitweise auch zur Beatmung, beispielsweise mit Überdruck, eines Patienten genutzt werden und zum Sekretmanagement umgeschaltet werden. In manchen Ausführungsformen kann der Unterdruck dem Schlauchsystem für eine Exsufflation bereitgestellt werden.

In manchen Ausführungsformen umfasst das System zumindest ein Therapiegerät, welches dazu ausgebildet ist mittels zumindest einer steuerbaren Quelle bzw. Senke für Unterdruck und/oder Überdruck eine Insufflation und/oder eine Exsufflation zu erzeugen. In manchen Ausführungsformen umfasst das Therapiegerät eine Quelle für Gas zur Erzeugung eines Überdrucks und eine Senke für Gas zur Erzeugung eines Unterdrucks. Es kann dabei vorgesehen sein, dass die Quelle und Senke kombiniert sind, die Quelle also gleichzeitig auch als Senke dienen kann. Das Therapiegerät ist also dazu ausgebildet einen Patienten beim Husten zu unterstützen und/oder ein Husten des Patienten herbeizuführen. Ziel des Hustens ist beispielsweise ein Sekretmanagement, unter anderem das Abhusten bzw. Entfernen von Sekret aus den Atemwegen (inklusive Lunge) des Patienten. Es kann auch vorgesehen sein, dass das Therapiegerät dazu ausgebildet ist, das Husten für den Patienten durchzuführen. Über ein Schaltmittel kann gesteuert werden, ob dem Patienten Überdruckgas oder Unterdruckgas zugeführt wird.

### Mechanismus zur Erinnerung an die Therapie

In manchen Ausführungsformen umfasst das System einen Mechanismus zu Erinnerung an die Therapie, den Therapieerinnerungsmechanismus. Der Erinnerungsmechanismus ist so gestaltet, dass auf Basis einer Datenlage eine Erinnerung an die Therapie erfolgt. Eine solche Erinnerung kann in verschiedenen Formen ausgebildet sein, welche optional miteinander kombinierbar sind.

Bei einer Form der Erinnerung wird beispielsweise durch das System eine Meldung und/oder ein Alarm generiert, welcher den Patienten und/oder Betreuer darauf hinweist, dass eine Therapie erfolgen sollte und/oder muss. Eine solche Meldung kann beispielsweise über eine Benutzerschnittstelle ausgegeben werden. Es kann dabei vorgesehen sein, dass die Meldung lediglich einen Hinweis enthält, dass eine Therapie durchgeführt werden soll. In manchen Ausführungsformen enthält die Meldung/der Alarm zusätzliche Informationen. Diese zusätzlichen Informationen können beispielsweise eine empfohlene Therapieform und/oder ein oder mehrere Gründe für die Therapie und/oder Hinweise zu den nächsten Schritten zur Durchführung der Therapie sein. Gründe für die Durchführung sind beispielsweise eine Zeitdauer seit der letzten Durchführung einer Therapie und/oder eine medizinische Relevanz, etwa Sekret-Akkumulation in den Atemwegen und/oder ein regelmäßiges Sekretmanagement. Darüber hinaus kann eine medizinische Relevanz gegeben sein, wenn eine Erkrankung, wie beispielsweise ein Infekt vorlag oder vorliegt. Des Weiteren könnten andere medizinische Messungen wie respiratorischer Fluss, SpO2, CO2, Blutdruck, Blutzuckerspiegel oder die Einnahme von Medikamenten die Erinnerung beeinflussen.

Die Hinweise zu den nächsten Schritten der Therapie können zum Beispiel eine Anleitung zur Durchführung enthalten. Optional wird diese Anleitung Schritt für Schritt zusammen mit den Aktionen des Patienten und/oder Betreuers durchgegangen. Sieht der aktuelle Anleitungsschritt etwa vor, dass ein Patienteninterface angeschlossen werden soll, so geht die Anleitung zum nächsten Schritt über, wenn erkannt wird, dass ein Patienteninterface angeschlossen wurde. Für Aktionen, welche das System nicht selbstständig erkennen kann, kann vorgesehen sein, dass eine Bestätigung über eine Benutzerschnittstelle vorgesehen ist.

Es kann alternativ oder ergänzend vorgesehen sein, dass die Auffälligkeit der Erinnerung abgestuft ist. Basiert die Erinnerung beispielsweise auf einer Zeitdauer seit der letzten Therapie, kann beim frühesten vorgesehenen Zeitpunkt für die Therapie eine einfache Erinnerung generiert werden, welche zum Beispiel einen Hinweis darauf enthält, dass eine gewisse Zeitdauer seit der letzten Therapie verstrichen ist und/oder dass eine Therapie durchgeführt werden kann bzw. sollte. Diese erste einfache Erinnerung kann beispielsweise durch den Patienten und/oder Anwender abgebrochen, ignoriert und/oder zeitlich verschoben werden. Nach einer weiteren Zeitdauer kann beispielsweise eine auffälligere Erinnerung generiert werden, sodass der Patient und/oder der Betreuer erkennen kann, dass die Durchführung der Therapie akuter wird, also die Therapie bald bzw. zeitnah durchgeführt werden sollte.

Es kann dabei vorgesehen sein, dass eine minimale und maximale Zeitdauer zwischen zwei Therapien vorgesehen ist. Beispielsweise kann vorgesehen sein, dass je näher die aktuelle Zeitdauer an der maximalen Zeitdauer liegt, desto auffälliger werden die Erinnerungen. Die Erinnerungen können beispielsweise durch eine farbliche Gestaltung, Schriftgröße, Animationen (z.B. Blinken der Anzeige) und/oder akustische Unterstützung auffälliger werden. Ist beispielsweise die maximale Zeitdauer seit der letzten Therapie erreicht, kann die Erinnerung in Form eines Alarms gestaltet sein, welcher deutlich wahrnehmbar ist, beispielsweise durch einen akustischen Alarm.

In manchen Ausführungsformen kann vorgesehen sein, dass die Erinnerungen zeitlich verschoben werden können. Wird eine Erinnerung durch den Mechanismus generiert, der Patient und/oder der Betreuer ist aber nicht bereit für eine Therapie, kann beispielsweise durch eine Bestätigung der Erinnerung die Erinnerung quittiert werden, sodass zu einem späteren Zeitpunkt erneut eine Erinnerung generiert wird. Es kann auch vorgesehen sein, dass beim Erreichen einer maximalen Zeitdauer seit der letzten Therapie die Erinnerung nicht weiter verschoben werden kann. Ist eine besondere Eskalationsstufe - etwa durch akustische Signale - vorgesehen, kann in manchen Ausführungsformen die Erinnerung lediglich stumm geschalten, aber nicht deaktiviert werden.

Eine Erinnerung an die Therapie ist zumindest auf dem Therapiegerät vorgesehen. Die Erinnerung kann aber auch auf weitere Geräte ausgeweitet werden. Beispielsweise kann eine Kopplung des Therapiegeräts mit einem Beatmungsgerät vorgesehen sein, bei der zumindest zeitweise Daten zwischen den Geräten ausgetauscht werden. Es kann dabei vorgesehen sein, dass die Erinnerung zusätzlich oder alternativ auf dem Beatmungsgerät generiert wird, der Patient und/oder der Betreuer also über das Beatmungsgerät an die Durchführung einer Therapie erinnert wird. Auch kann eine Erinnerung über externe Gegenstellen, etwa eine App auf einem mobilen Endgerät und/oder einem Computer, beispielsweise mit einer Cloud verbunden, generiert und/oder angezeigt werden.

In manchen Ausführungsformen wird der Patient und/oder Betreuer auf die Erinnerung folgend und/oder zusammen mit der Erinnerung aufgefordert Einstellungen zur Therapie zu wählen. Es kann dabei vorgesehen sein, dass zumindest der Start der Therapie gewählt werden kann. Alternativ oder ergänzend wird der Patient und/oder Benutzer aufgefordert die Therapieform zu wählen und/oder verschiedene Parameter zur Therapie einzugeben und/oder zu wählen.

### Mechanismus zum Start der Therapie

In manchen Ausführungsformen umfasst das System alternativ oder ergänzend einen Mechanismus zum Start der Therapie, den Therapiestartmechanismus. Der Startmechanismus ist so ausgestaltet, dass auf Basis einer Datenlage ein Start der Therapie erfolgt. Ein solcher Start kann in verschiedenen Formen ausgebildet sein, welche optional miteinander kombinierbar sind.

Eine Form des Therapiestarts ist eine Vorbereitung der Therapie. Erkennt das System beispielsweise anhand der Datenlage, dass ein Therapiestart erfolgen soll, kann ein Start im Sinne einer Vorbereitung der Therapie erfolgen. Die Vorbereitung umfasst beispielsweise das Laden eines Therapieprogramms, welches zum Beispiel eine Anzahl an Manövern und/oder Zyklen beinhaltet. Auch andere Parameter zur Therapie, etwa die Intensität der Therapie, kann in einem Therapieprogramm hinterlegt sein. Alternativ oder ergänzend umfasst die Vorbereitung der Therapie das Bereitmachen des Therapiegerätes, beispielsweise durch Hochfahren und/oder Aufwärmen. Eine Ausführungsform der Vorbereitung kann auch dadurch ausgeprägt sein, dass lediglich eine Startmöglichkeit für den Patienten und/oder Betreuer gegeben wird. Beispielsweise kann ein Start-Knopf freigegeben werden und/oder auf einem berührungsempfindlichen Bildschirm (Touchscreen) angezeigt werden. In manchen Ausführungsformen kann auch vorgesehen sein, dass das System durch den Therapiestartmechanismus vorbereitet wird und die Durchführung der Therapie über eine entfernte Gegenstelle, zum Beispiel eine App auf einem mobilen Endgerät, gestartet wird.

Zusätzlich kann auch vorgesehen sein, dass die Vorbereitung der Therapie eine Anzeige von durchzuführenden Schritten für die Therapie anzeigt. Diese Schritte können beispielsweise das Wechseln eines Patienteninterfaces und/oder die Eingabe bzw. Abfrage von zusätzlichen Angaben/Daten/Informationen/Parametern zur Therapie sein.

Der Startmechanismus kann alternativ oder ergänzend auch vorsehen, dass System in einen Zustand versetzt wird, bei dem der Patient und/oder Betreuer nur einen Start-Knopf betätigen muss, damit die Therapie durchgeführt wird.

In manchen Ausführungsformen ist in dem System ein Therapiegerät mit einem Beatmungsgerät so gekoppelt, dass zumindest ein Datenaustausch stattfindet. Es kann beispielhaft vorgesehen sein, dass in Zeiten, in denen das Therapiegerät nicht genutzt wird, eine Anzeige der Benutzerschnittstelle des Therapiegeräts dazu genutzt wird um eine Anzeige des Beatmungsgeräts zu erweitern. Der Therapiestartmechanismus kann dabei verschiedene Auswirkungen auf diese Kopplung haben.

Beispielsweise kann vorgesehen sein, dass der Startmechanismus zum Start der Therapie zumindest eine erweiterte Anzeige beendet. Alternativ oder ergänzend kann auch vorgesehen sein, dass eine zusätzliche Darstellung bezüglich des Therapiestarts auf der Anzeige des Beatmungsgerätes generiert wird. Wird die erweiterte Anzeige des Beatmungsgerätes durch den Startmechanismus beendet, kann vorgesehen sein, dass auf der Anzeige des Therapiegerätes Darstellungen entsprechend des Therapiestarts angezeigt werden. Bei einer Kopplung von Therapiegerät mit Beatmungsgerät in einer Form, dass zumindest Daten ausgetauscht werden, kann auch vorgesehen sein, dass Daten des Beatmungsgerätes mit in die Datenlage, welche als Entscheidungsbasis für den Therapiestartmechanismus dient, miteinbezogen werden können.

In manchen Ausführungsformen des Systems ist das Therapiegerät alternativ oder ergänzend pneumatisch mit einem Beatmungsgerät gekoppelt. Eine pneumatische Kopplung kann dabei vorsehen, dass zur Durchführung lediglich das Patienteninterface gewechselt werden muss. Es kann auch vorgesehen sein, dass ein Patienteninterface verwendet wird, welches sowohl für die Beatmung als auch für eine Hustentherapie geeignet ist. Es kann also vorgesehen sein, dass der Therapiestartmechanismus die Durchführung der Therapie automatisch startet.

Bei einer pneumatischen Kopplung kann beispielsweise ein Ventil vorgesehen sein, welches zwischen der Pneumatik des Beatmungsgerätes und der Pneumatik des Therapiegerätes umschaltet. Entscheidet der Therapiestartmechanismus auf Basis der Datenlage, dass eine Therapie durchgeführt werden soll, kann beispielsweise vorgesehen sein, dass die Therapie automatisch gestartet wird. Dabei wird beispielsweise über das Ventil von der Pneumatik des Beatmungsgeräts auf die Pneumatik des Therapiegerätes umgeschaltet, sodass die Therapie durchgeführt werden kann. Der Therapiestart kann also ohne Zutun des Patienten und/oder eines Betreuers gestartet werden.

Alternativ oder ergänzend kann vorgesehen sein, dass über das Therapiegerät und/oder das Beatmungsgerät eine Meldung generiert wird, welche den Betreuer und/oder den Patienten auf den anstehenden Therapiestart hinweist. Es kann beispielsweise vorgesehen sein, dass der Patient und/oder Betreuer durch visuelle Hinweise aufmerksam gemacht wird. Auch kann vorgesehen sein, dass der Patient und/oder Betreuer durch ein Audiosignal auf den bevorstehenden Therapiestart hingewiesen wird. Optional können auch mehrere Hinweise erfolgen, beispielsweise in bestimmten Zeitabständen vor Beginn der Therapie. Es kann beispielsweise vorgesehen sein, dass in einem gewissen zeitlichen Abstand zum Therapiestart über visuelle Hinweise auf die Therapie aufmerksam gemacht wird. Kommt der Therapiestart näher, so können die visuellen Hinweise durch Audiosignale unterstützt werden. Dadurch wird der Patient und/oder Betreuer nicht von einer einsetzenden Therapie überrascht. Der automatische Start der Therapie kann auch eine automatische Auswahl eines Therapieprogramms umfassen.

Wird auf Basis der Datenlage vom System festgestellt, dass eine akute Situation vorherrscht, bei der ein sofortiger Therapiestart sinnvoll ist, kann auch vorgesehen sein, dass die Therapie ohne weitere Hinweise gestartet wird. In manchen Ausführungsformen kann vorgesehen sein, dass zusätzlich auch eine Alarm-Meldung generiert wird, welche auf die akute Situation hinweist, etwa über eine entfernte Gegenstelle wie einem verbundenen Server und/oder einem mobilen Endgerät.

In manchen Ausführungsformen umfasst das System ein kombiniertes Beatmungs- und Therapiegerät. Der Mechanismus zum Starten der Therapie kann dabei wie bei einer pneumatischen Kopplung eines Therapiegerätes und eines Beatmungsgerätes wirken. Dabei wird in dem kombinierten Gerät zum Starten der Therapie zwischen einem Beatmungsmodus und einem Therapiemodus umgeschaltet.

Alternativ oder ergänzend kann auch eine Kombination des Therapiestartmechanismus mit einem Erinnerungsmechanismus vorgesehen sein. Je nach Datenlage kann beispielsweise zunächst eine Erinnerung an die Therapie eingeleitet werden bevor ein Start der Therapie erfolgt. Beispielhaft wird zumindest einmal an die Therapie erinnert. Ignoriert der Nutzer die Erinnerung und/oder wird ein Zeitpunkt und/oder eine Datenlage erreicht, dass ein Beginn der Therapie nötig ist, kann vorgesehen sein, dass von dem Erinnerungsmechanismus auf den Startmechanismus umgeschaltet wird. Der Startmechanismus kann beispielsweise einen Hinweis generieren, dass nach einer Zeitspanne ein Therapiestart erfolgt. Nach Ablauf dieser Zeitspanne startet die Therapie entsprechend des beschriebenen Therapiestartmechanismus. Bei pneumatisch gekoppelten Beatmungs- und Therapiegeräten und kombinierten Therapie-/Beatmungsgeräten kann beispielsweise die Durchführung der Therapie automatisch beginnen. Bei nicht-gekoppelten oder nur für einen Datenaustausch gekoppelten Geräten kann hingegen vorgesehen sein, dass der Therapiestart die Vorbereitung der Therapie, z.B. Aufwärmen/Hochfahren des Gerätes/einzelner Komponenten, betrifft.

### Mechanismus zum Wiederholen der Therapie

In manchen Ausführungen umfasst das System alternativ oder ergänzend einen Mechanismus zum Wiederholen der Therapie, den Therapiewiederholungsmechanismus. Der Wiederholungsmechanismus entscheidet auf Basis einer Datenlage, ob die Therapie wiederholt und/oder verlängert wird. Die Datenlage kann beispielsweise Daten umfassen, welche während einer aktuell durchgeführten Therapie erfasst werden. Es kann vorgesehen sein, dass zur Entscheidung der Therapiewiederholung und/oder Therapieverlängerung bewertet wird, ob und/oder in welchem Umfang die Therapie eine Wirkung erzielt. Es kann zum Beispiel erkannt werden, dass der Patient zu wenige oder keine Hustenstöße zeigt und/oder andere Daten auf eine unzureichende Therapie hinweisen. Eine unzureichende Therapie liegt zum Beispiel dann vor, wenn der Peak Cough Flow (PCF) zu niedrig war und/oder bestimmte Drücke nicht erreicht wurden. In manchen Ausführungsformen kann dies alternativ oder ergänzend auch auf eine (zu hohe) Leckage oder eine zu geringe Sauerstoffsättigung (SpO2) hinweisen. Es kann in der Folge vorgesehen sein, dass in Form einer Therapieverlängerung zusätzliche Hustenmanöver und/oder Hustenzyklen der aktuellen Therapie hinzugefügt werden. Beispielsweise werden so lange Zyklen und/oder Manöver hinzugefügt, bis ein bestimmter Schwellenwert an Hustenstößen des Patienten und/oder anderer Daten erreicht wird, welche auf eine mindestens ausreichende Therapie hinweisen. Optional kann auch vorgesehen sein, dass die zusätzlichen Manöver und/oder Zyklen angepasste Parameter aufweisen. Wenn die erfassten Daten während der aktuellen Therapie beispielsweise darauf hinweisen, dass die Intensität der Zyklen und/oder Manöver zu gering ist um ein Husten zu erzeugen, beispielsweise um durch den Wechsel zwischen Überdruck und Unterdruck eine Reaktion des Patienten auszulösen, kann vorgesehen sein, dass die nachfolgenden und/oder hinzugefügten Zyklen und/oder Manöver eine entsprechend angepasste Intensität aufweisen.

Alternativ oder ergänzend kann auch eine Wiederholung der Therapie vorgesehen sein. Beispielsweise wird im Anschluss, optional mit einer einstellbaren, festen und/oder automatisch ermittelten zeitlichen Verzögerung die gleiche Therapie erneut durchgeführt werden. In manchen Ausführungsformen kann auch vorgesehen sein, dass statt der gleichen Therapie eine zumindest teilweise geänderte Therapie als Wiederholung durchgeführt wird. Beispielsweise können die Änderungen zumindest teilweise auf Daten, welche während der ursprünglichen Therapie erfasst werden, basieren.

In manchen Ausführungsformen kann ein Therapiekombinationsmechanismus vorgesehen sein, welcher die Funktionen von zumindest zwei der vorgenannten Mechanismen kombiniert. Beispielsweise werden in dem Kombinationsmechanismus die Funktionen des Erinnerungsmechanismus und des Startmechanismus kombiniert. Auch eine Kombination von Erinnerungs- und Wiederholungsmechanismus und/oder eine Kombination von Wiederholungs- und Startmechanismus sind denkbar. In manchen Ausführungsformen umfasst der Therapiekombinationsmechanismus die Funktionen des Therapieerinnerungs-, Therapiestart- und Therapiewiederholungsmechanismus.

### Kopplung von Therapiegerät und Beatmungsgerät

In manchen Ausführungsformen umfasst das System neben einem Therapiegerät auch ein Beatmungsgerät, welcher mit dem Therapiegerät gekoppelt ist. Es kann eine Kopplung zum Datenaustausch zwischen den Geräten und/oder eine pneumatische Kopplung der Geräte vorgesehen sein.

Bei einer Kopplung der Geräte zum Datenaustausch sind das Therapiegerät und das Beatmungsgerät über eine Datenleitung miteinander verbunden. Diese Datenleitung kann eine kabelgebundene Verbindung und/oder eine kabellose Verbindung sein. Durch den Datenaustausch können beide Geräte auf die Daten, welche von dem jeweils anderen Gerät erfasst werden, zugreifen. Beispielsweise wird ein Beatmungsgerät über einen größeren Zeitraum genutzt als das Therapiegerät und kann somit auch eine umfangreichere Menge an Daten erfassen. Insbesondere erfasst das Beatmungsgerät Daten während der Beatmung eines Patienten. Diese Daten können beispielsweise auch dahingehend ausgewertet werden, ob eine Therapie mit dem Therapiegerät sinnvoll ist und/oder durchgeführt werden sollte. In manchen Ausführungsformen ist das Therapiegerät dazu eingerichtet, dass bei einer Kopplung mit einem Beatmungsgerät die Anzeige des Beatmungsgerätes zumindest zeitweise über die Anzeige der Benutzerschnittstelle des Therapiegerätes erweitert werden kann.

Bei einer Kopplung zum Datenaustausch kann auch vorgesehen sein, dass manche Berechnungen und/oder Auswertungen von Daten des Beatmungsgerätes auf dem Therapiegerät durchgeführt werden. Beispielsweise kann eine Auswertung der Daten zur Beurteilung, ob eine Therapie notwendig ist, auf dem Therapiegerät durchgeführt werden. In manchen Ausführungsformen kann das Beatmungsgerät so eingerichtet sein, dass es selbständig eine Auswertung zur Beurteilung der Notwendigkeit einer Therapie durchführt und ein entsprechendes Ergebnis an das Therapiegerät weiterleitet. Die Daten des Beatmungsgerätes können alternativ oder ergänzend auch vom Therapiegerät genutzt werden um Parameter der Therapie anzupassen und/oder eine Therapieform bzw. ein Therapieprogramm auszuwählen.

In manchen Ausführungsformen ist vorgesehen, dass Daten, welche vom Therapiegerät während der Therapie erfasst werden zumindest teilweise dahingehend ausgewertet werden, ob und/oder wie die Beatmung mit dem Beatmungsgerät angepasst werden kann. Die Auswertung kann dazu auf dem Therapiegerät und/oder dem Beatmungsgerät stattfinden. Die vom Beatmungsgerät erfassten Daten können also die Therapie beeinflussen und/oder die vom Therapiegerät erfassten Daten können die Beatmung beeinflussen.

In manchen Ausführungsformen des Systems ist alternativ oder ergänzend ein Therapiegerät pneumatisch mit einem Beatmungsgerät gekoppelt. Die pneumatische Kopplung kann unabhängig von oder gemeinsam mit einer Kopplung zum Datenaustausch erfolgen. Bei einer pneumatischen Kopplung kann beispielsweise vorgesehen sein, dass das Schlauchsystem zwischen Patient und Gerät nicht getauscht werden muss, sondern zwischen der Pneumatik des Beatmungsgerätes und der Pneumatik des Therapiegeräts umgeschaltet wird. Beispielsweise ist eine Ventileinrichtung mit beiden Pneumatiken verbunden und kann durch eine Steuereinheit gesteuert werden um zwischen Beatmung und Therapie umzuschalten. Alternativ kann auch ein kombiniertes Therapie- und Beatmungsgerät vorgesehen sein. Ein kombiniertes Gerät umfasst beispielsweise getrennte Einrichtungen zur Durchführung der Therapie und zur Beatmung und/oder eine Einrichtung, welche sowohl die Therapie als auch eine Beatmung leisten kann.

### Datenlage als Basis

Als Basis für den Mechanismus zur Therapieerinnerung und/oder zum Therapiestart und/oder zur Therapiewiederholung dient eine Datenlage. Die Datenlage kann mehrere Daten und Parameter umfassen.

In manchen Ausführungsformen umfasst die Datenlage, welche als Basis für zumindest einen der Mechanismen (Therapieerinnerung, Therapiestart, Therapiewiederholung) dient, zumindest eine Zeitdauer. Die Zeitdauer kann zum Beispiel eine Zeitdauer seit der letzten Therapie sein. Wie die Zeitdauer bewertet wird kann beispielsweise fest vorgegeben sein und/oder durch den Patienten und/oder Betreuer einstellbar sein und/oder anhand der letzten und/oder folgenden Therapie bestimmt werden.

Eine (optional wiederkehrende) Therapie kann beispielsweise ein regelmäßiges Sekretmanagement sein, welche regelmäßig durchgeführt wird um sich ansammelndes Sekret aus den Atemwegen eines Patienten zu entfernen. Bei einem gesunden Menschen passiert dies für gewöhnlich fast unbemerkt und/oder ohne Hilfe beispielsweise durch tiefes Einatmen und/oder selbständiges Husten. Bei manchen Menschen ist es hingegen notwendig dieses Sekretmanagement zu unterstützen oder mandatorisch durchzuführen. Das Sekretmanagement kann dabei beispielhaft eine Insufflation mit nachfolgendem Husten, eine kombinierte Insufflation/Exsufflation und/oder das Absaugen von Sekret aus den Atemwegen umfassen. Zwischen den Therapien kann eine Zeitdauer vorgesehen sein, nach welcher die Therapie erneut durchgeführt wird. Der jeweilige Mechanismus, beispielsweise der Therapieerinnerungsmechanismus und/oder der Therapiestartmechanismus, können dann beispielsweise auf eine vergangene Zeitdauer reagieren und die Erinnerung und/oder den Therapiestart aktivieren. Alternativ oder ergänzend kann auch ein Zeitplan vorgesehen bzw. einstellbar sein, welcher zwischen den Therapien optional eine jeweils unterschiedliche Zeitdauer vorsieht. Ein solcher Zeitplan kann etwa vorsehen, dass während einer Wachphase des Patienten eine kürzere Zeitdauer zwischen den Therapien vorgesehen ist und während der Schlafphase eine längere Zeitdauer bzw. die Zeitdauer so gewählt wird, dass während der Schlafphase keine Therapie stattfinden soll.

Die Datenlage kann alternativ oder ergänzend auch einen Therapieplan umfassen, in welchem Zeitpunkte definiert sind, zu denen eine Therapieerinnerung und/oder ein Therapiestart ausgelöst wird. Auch kann vorgesehen sein, dass in dem Therapieplan verschiedene Therapieformen hinterlegt sind, welche zu bestimmten Zeitpunkten vorgesehen sind. Der definierte Zeitpunkt kann in manchen Ausführungsformen auch ein Zeitraum sein, innerhalb welchem die Therapie durchgeführt werden sollte. Beispielsweise kann für den Therapieerinnerungsmechanismus vorgesehen sein, dass zu Beginn des Zeitraumes bzw. am Zeitpunkt eine erste Erinnerung generiert wird. Bei einer kombinierten Ausführung des Erinnerungsmechanismus und des Startmechanismus kann vorgesehen sein, dass zum definierten Zeitpunkt zunächst eine Erinnerung erzeugt wird und nach einer definierten und/oder einstellbaren Anzahl an Erinnerungen ein Therapiestart ausgelöst wird.

In manchen Ausführungsformen umfasst die Datenlage alternativ oder ergänzend Daten und/oder Informationen, welche von einem Patienten und/oder Betreuer beispielsweise über eine Benutzerschnittstelle eingegeben werden. Diese Daten und/oder Informationen können beispielsweise Patientendaten wie Geschlecht, Alter, Größe, Gewicht und/oder Erkrankungen sein. In manchen Ausführungsformen kann vorgesehen sein, dass der Therapieerinnerungsmechanismus und/oder der Therapiestartmechanismus und/oder der Therapiewiederholungsmechanismus anhand der eingegebenen Daten und/oder Informationen eine Zeitdauer bestimmt und/oder empfohlen wird, welche zwischen zwei Therapien liegt. Auch kann vorgesehen sein, dass die Therapieform durch den jeweiligen Mechanismus bestimmt wird und/oder eine Empfehlung zur Therapieform erzeugt wird. Ferner kann vorgesehen sein, dass ein Patient und/oder Betreuer zwischen zwei Therapien zusätzliche Informationen und/oder Daten eingeben kann, welche für die nächsten Zeitdauern berücksichtigt werden. Beispielsweise kann ein Infekt beim Patienten festgestellt werden und dies als Information zwischen zwei Therapien eingegeben werden, sodass beispielsweise die Zeitdauer zwischen den Therapien und/oder die Therapieform bzw. Therapieintensität angepasst wird und/oder eine Empfehlung zur Anpassung generiert wird.

Wird beispielsweise eingegeben, dass der Patient an einem Infekt leidet, kann vorgesehen sein, dass die Zeitdauer zwischen zwei Therapien verkürzt wird. Zusammenhängen damit kann optional vorgesehen werden, dass zusätzlich weitere Einstellungsmöglichkeiten mit Bezug zur Therapie angeboten werden. In manchen Ausführungsformen können verschiedene Arten und/oder Schwere von Infekten angegeben werden, worauf der jeweilige Mechanismus unterschiedliche Änderungen der Zeitdauer und/oder anderer Therapieparameter vornimmt und/oder anbietet.

In manchen Ausführungsformen umfasst die Datenlage zusätzlich oder alternativ auch von dem Therapiegerät erfasste Daten. Die von dem Therapiegerät erfassten Daten können zum Beispiel Beatmungsdaten und/oder Therapiedaten sein. Während der Therapie können beispielsweis vom Therapiegerät Daten wie zum Beispiel Sensordaten erfasst und ausgewertet werden. Darunter fallen beispielsweise zumindest Fluss und/oder Druck und/oder eine Frequenz, beispielsweise Atem- und/oder Hustenfrequenz. Es kann zudem vorgesehen sein, dass das Therapiegerät dazu ausgebildet ist, die erfassten Daten auszuwerten. Anhand der Auswertung der Daten kann der Erinnerungsmechanismus und/oder der Startmechanismus und/oder der Wiederholungsmechanismus entscheiden, wann und/oder ob eine Therapie vorgesehen ist und/oder wiederholt und/oder verlängert wird. Beispielsweise kann vorgesehen sein, dass eine Live-Auswertung der Daten erfolgt und der Therapiewiederholungsmechanismus anhand der Auswertung entscheiden kann, ob eine Wiederholung oder Verlängerung der Therapie nötig ist. Es kann auch vorgesehen sein, dass der Therapiewiederholungsmechanismus alternativ oder ergänzend eine Empfehlung zur Wiederholung und/oder Verlängerung ausgibt, welche durch den Patienten und/oder Betreuer übernommen und/oder abgeändert werden kann. Auch kann vorgesehen sein, dass der Therapieerinnerungsmechanismus und/oder der Therapiestartmechanismus anhand der während der Therapie erfassten Daten, optional in Kombination mit weiteren Daten und Informationen, die Zeitdauer bis zur nächsten Therapie anpassen kann. Auch kann vorgesehen sein, dass die Therapieform anhand der erfassten Daten angepasst wird.

Wird bei der Auswertung der Daten, beispielsweise durch eine Auswerteeinheit und/oder einem der Mechanismen, erkannt, dass eine unzureichende Anzahl und/oder Intensität an Hustenstößen erfolgt, kann vorgesehen sein, dass der Therapiewiederholungsmechanismus eine Verlängerung und/oder Wiederholung der Therapie vorsieht. Es kann dabei auch vorgesehen sein, dass der Wiederholungsmechanismus keine unmittelbare Wiederholung der Therapie vorsieht, sondern eine zeitlich verzögerte Wiederholung. Alternativ oder ergänzend kann vorgesehen sein, dass der Wiederholungsmechanismus eine Verkürzung der Zeitdauer bis zur nächsten Therapie bestimmt bzw. einstellt. Für den Therapieerinnerungsmechanismus und/oder den Therapiestartmechanismus kann vorgesehen sein, dass bei unzureichenden Hustenstößen, die Zeitdauer bis zur nächsten Therapie verkürzt wird und/oder die Therapieform bzw. Therapieintensität angepasst wird.

Beispielsweise kann vorgesehen sein, dass der Hustenfluss erfasst wird, insbesondere die Spitzenwerte des Hustenflusses (PCF, Peak Cough Flow). Der PCF kann beispielsweise Hinweise dazu liefern, ob ein Hustenmanöver und/oder ein Hustenzyklus effektiv war. Beispielsweise kann der Therapiewiederholungsmechanismus anhand des PCF entscheiden, ob das Husten zu schwach war und die Therapie wiederholt und/oder verlängert wird. Bei einer Verlängerung werden beispielsweise weitere Hustenmanöver und/oder Zyklen an die aktuelle Therapie angehängt.

In manchen Ausführungsformen kann alternativ oder zusätzlich vorgesehen sein, dass die Datenlage vom Beatmungsgerät erfasste Daten umfasst. Dazu können beispielsweise Sensordaten und Werte zählen, welche vom Beatmungsgerät während der Beatmung erfasst werden. Die Berücksichtigung von Daten des Beatmungsgerätes kann insbesondere bei einer Kopplung zur Datenübermittlung zwischen Therapiegerät und Beatmungsgerät erfolgen. Anhand der vom Beatmungsgerät erfassten Daten kann beispielsweise ausgewertet werden, ob eine Therapie nötig ist. Beispielsweise können die Beatmungsdaten wie Fluss und/oder Druck darauf hinweisen, dass sich eine gewisse Menge an Sekret in den Atemwegen des Patienten angesammelt hat und durch eine Therapie entfernt werden sollte. In manchen Ausführungsformen können die Beatmungsdaten auch auf eine zunehmende Verschlechterung und/oder Verbesserung der Ansammlung von Sekret beim Patienten hinweisen. In solchen Fällen kann beispielsweise vorgesehen sein, dass die Zeitdauer bis zur nächsten Therapieerinnerung und/oder Therapiestart automatisch und/oder dynamisch angepasst wird. Auch kann dabei vorgesehen sein, dass die Therapieform und/oder Therapieintensität angepasst wird.

Es kann beispielsweise vorgesehen sein, dass ein SpO2-Wert und/oder ein CO2-Wert und/oder andere Blutgaswerte und/oder andere Atemgaswerte erfasst vom Beatmungsgerät erfasst werden. Anhand einer Auswertung dieser Werte kann beispielsweise festgestellt werden, dass ein Husten nötig ist. Eine beispielhafte Herangehensweise ist die Methode nach John R. Bach. Dabei wird berücksichtigt, dass ein Abfall des SpO2-Wertes unter einen Schwellenwert bei bestimmten Patiententypen, beispielsweise mit Vorerkrankung, auf eine Ansammlung von Sekret hindeutet, ein Abhusten also sinnvoll und/oder nötig ist. Der Schwellenwert liegt beispielsweise zwischen 90% und 98%, bevorzugt zwischen 93,5% und 96%. Beispielsweise liegt der Schwellenwert bei 95% oder 94%.

Ergänzend oder alternativ kann ein Signal-Rausch-Verhältnis (SNR, Signal to Noise Ratio), beispielsweise des Druck- und/oder Flusssignals, erfasst und/oder ausgewertet werden. Es kann beispielsweise vorgesehen sein, dass bei einem hohen SNR auf eine größere Menge an Sekret geschlossen wird. Dies wiederum führt dazu, dass das Zeitintervall bis zur nächsten Erinnerung und/oder Durchführung verkürzt wird.

Ergänzend oder alternativ können Schwankungen im Druck und/oder Fluss erfasst und/oder ausgewertet werden. Die erfassten Schwankungen können unter anderem beispielsweise durch Strömungsabrisse und/oder Verschlüssen/Wiederöffnungen resultieren und auf eine größere Menge an Sekret deuten. Diese Auswertung kann beispielsweis zu einer Verkürzung des Zeitintervalls bis zur nächsten Erinnerung und/oder Durchführung führen.

In manchen Ausführungsformen können alternativ oder ergänzend auch akustische Signale und/oder Töne erfasst und ausgewertet werden. So kann beispielsweise erkannt werden, ob sich Sekret oder andere Flüssigkeiten in der Lunge des Patienten angesammelt haben. Beispielhaft können die Atemgeräusche des Patienten erfasst und ausgewertet werden.

In manchen Ausführungsformen werden mehrere Datenquellen genutzt. Es kann beispielsweise vorgesehen sein, dass ein Therapiedringlichkeitsindex bestimmt wird. Der Dringlichkeitsindex kann beispielsweise aus einer Zeitdauer seit der letzten Therapie und weiteren Daten berechnet werden. Eine Therapieerinnerung und/oder ein Therapiestart wird beispielsweise dann ausgelöst, wenn der Dringlichkeitsindex einen definierten, optional einstellbaren, Schwellenwert erreicht. Beispielsweise kann vorgesehen sein, dass der Dringlichkeitsindex mit höherer Zeitdauer seit der letzten Therapie steigt. Je nach Auswertung weiterer Daten kann der Dringlichkeitsindex zusätzlich erhöht werden. Ergibt die Auswertung von Daten beispielsweise, dass sich zunehmend Sekret in den Atemwegen des Patienten ansammeln, kann dadurch eine zusätzliche Erhöhung des Dringlichkeitsindex erfolgen. Optional kann auch vorgesehen sein, dass anhand der Datenlage ein Grundwert des Dringlichkeitsindex bestimmt wird. Liegt etwa ein Infekt des Patienten vor, kann vorgesehen sein, dass der Grundwert des Dringlichkeitsindexes höher ist als ohne Infekt. In der Folge ist der Schwellenwert nach einer kürzeren Zeitdauer erreicht.

In manchen Ausführungsformen ist vorgesehen, dass das Therapiegerät dazu eingerichtet ist eine Langzeitprognose und/oder Langzeitauswertung durchführen zu können. Über eine solche Prognose und/oder Auswertung kann beispielsweise eine höhere Genauigkeit des Therapiebedarfs eines Patienten erreicht werden. Beispielsweise wird dazu ausgewertet, wie sich verschiedene Therapieformen, Therapieparameter und/oder Therapieintensitäten auf die Entwicklung des Patienten auswirken. Es kann etwa vorgesehen sein, dass durch die Prognose und/oder Auswertung festgestellt wird, dass bestimmte Therapieparameter einen positiven Einfluss auf den Patienten haben, beispielsweise so, dass eine Therapie seltener nötig ist.

In manchen Ausführungsformen ist alternativ oder ergänzend vorgesehen, dass vom Therapiegerät und/oder Beatmungsgerät erfasst Daten in ein cloudbasiertes System hochgeladen werden. Das cloudbasierte System ist beispielsweise dazu eingerichtet, über Algorithmen therapiebezogene Entscheidungen zu treffen, beispielsweise was die Häufigkeit, Form und/oder Intensität der Therapie betrifft. Diese Entscheidungen werden wiederum aus der Cloud auf das Therapiegerät und/oder Beatmungsgerät geladen, wobei auf dem Therapiegerät und/oder dem Beatmungsgerät eine Anpassung der Therapieparameter (z.B. Häufigkeit, Form, Intensität) anhand der Entscheidungen durchgeführt wird. Die therapiebezogenen Entscheidungen können entsprechend auch Anpassungen des Therapiestart-, Therapieerinnerungs- und/oder Therapiewiederholungsmechanismus bewirken. Beispielsweise kann der Startzeitpunkt der Therapie angepasst werden und/oder die Zeitdauer zwischen zwei Therapien.

Offenbarungsgemäß wird anhand einer Datenlage eine Therapieerinnerung und/oder ein Therapiestart und/oder eine Therapiewiederholung durchgeführt. Dabei können verschiedene Daten genutzt werden. Beispielsweise kann eine Therapieerinnerung primär auf einer Zeitdauer, etwa seit der letzten Therapie, basieren und eine Therapiewiederholung auf Daten während der aktuellen und/oder letzten Therapie, optional ergänzt um eingegebene Daten, wie etwa Patientenparameter. Ein Therapiestart kann beispielsweise auch aufgrund von Beatmungsdaten ausgelöst werden, unter anderem wenn diese darauf deuten, dass ein Abhusten sinnvoll und/oder nötig erscheint. Aber auch ein Therapiestart kann ausschließlich aufgrund einer Zeitdauer ausgelöst werden.

In manchen Ausführungsformen ist alternativ oder ergänzend vorgesehen, dass zumindest der Therapieerinnerungsmechanismus eine Art Weckfunktion umfasst, wobei anhand einer Datenlage eine Erinnerung über das Therapiegerät und/oder ein optional gekoppeltes Beatmungsgerät und/oder eine entfernte Gegenstelle ausgegeben wird. Optional können mehrere Erinnerungsstufen vorgesehen sein, optional auch mit eskalierenden Hinweisen in Form von Bild und/oder Ton. In manchen Ausführungsformen kann die Erinnerung dem Patienten und/oder Betreuer auch eine Wahlmöglichkeit geben, um zum Beispiel Therapieform und/oder Therapieintensität und/oder eine Zeit bis zur nächsten Erinnerung auszuwählen.

In manchen Ausführungsformen kann alternativ oder ergänzend vorgesehen, dass die Erinnerungen des Therapieerinnerungsmechanismus über einen längeren Zeitraum (mehrere Tage/Wochen/Monate) unauffälliger werden. Ziel kann beispielsweise sein, dass der Patient an eine wiederkehrende Therapie gewöhnt wird und beginnt selbständig die Therapie regelmäßig zu starten. Optional kann vorgesehen sein, dass die Auffälligkeit der Erinnerungen zurückgesetzt wird und/oder wieder erhöht wird, sollte der Patient und/oder der Betreuer die Therapie ein- und/oder mehrmalig vergessen.

Die Kopplung der von Therapiegerät und Beatmungsgerät bringt verschiedene Möglichkeiten. Es kann ein reiner Datenaustausch stattfinden, jedes Gerät interpretiert die Daten für sich alleine. Beim reinen Datenaustausch kann auch vorgesehen sein, dass Erinnerungen etc. auf dem Beatmungsgerät angezeigt werden. Neben einer "Kabelkopplung" kann auch eine Kopplung per Funk/Bluetooth/WLAN vorgesehen sein. Auch kann eine Steuerung des Therapiegerätes über das Beatmungsgerät vorgesehen sein, beispielsweise ein Start/Stopp der Therapie. Zusätzlich ist auch eine gegenseitige Beeinflussung denkbar, Therapieergebnisse/-erkenntnisse können zu Änderung der Beatmung führen und umgekehrt kann die Therapie anhand von Beatmungsdaten (Patienten- und Geräte-bezogen) angepasst werden. Alternativ oder ergänzend kann auch vorgesehen sein, dass während der Beatmung durch das Beatmungsgerät, also in der Zeit in der das Therapiegerät nicht durch den Patienten genutzt wird, die Rechenleistung des Therapiegeräts genutzt werden kann um Auswertungen und/oder Analyse etc. des Beatmungsgerätes durchzuführen.

Auf Therapieerinnerung kann ein Therapiestart folgen. Nach Therapiestart ist eine Therapiewiederholung möglich. Bspw. nach einer Zeitdauer wird so lange erinnert bis ein Schwellenwert an Zeitdauer erreicht ist. Dann wird auf Therapiestart entschieden. Der "gezwungene" Therapiestart kann verschiedene Arten haben, welche eher bei gekoppelten Geräten sinnvoll sind. Beispielsweise kann bei pneumatisch gekoppelten Geräten nach einiger Zeit - nach Ankündigung - die Therapie einfach gestartet werden, ohne dass der Patient dies explizit anfordert. Die Therapieparameter können sich dabei je nach Datenlage einstellen, wobei auch unterschieden wird, ob ein erzwungener Start vorliegt oder der Patient die Therapie selber startet.

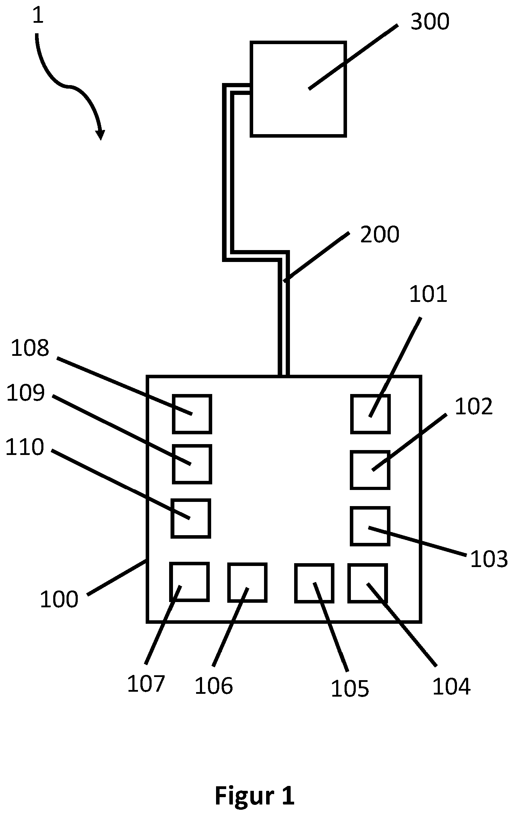

Die Offenbarung wird anhand der Figuren 1 bis 3 beispielhaft näher beschrieben. Figur 1 zeigt ein System 1 mit einem Therapiegerät 100, welches über ein Schlauchsystem 200 mit einem Patienten 300 verbunden ist.

Das Therapiegerät 100 umfasst zumindest eine steuerbare Quelle für Gas zur Erzeugung eines Überdrucks. Das System 1 umfasst ferner Schaltmittel 102, über welche das Schlauchsystem 200 mit Überdruck beaufschlagt werden kann, beispielsweise um eine Insufflation durchzuführen. Das System 1 ist mit den Schaltmitteln 102 dazu eingerichtet über die Beaufschlagung des Schlauchsystems 200 mit Überdruck plötzlich zu stoppen und eine Exspiration und/oder ein Husten des Patienten 300 zu forcieren.

In manchen Ausführungsformen umfasst das Therapiegerät 100 zumindest eine steuerbare Druckgasquelle zur Erzeugung eines Überdruckgases und eine Druckgasquelle zur Erzeugung eines Unterdruckgases bzw. zumindest eine steuerbare Quelle für Gas zur Erzeugung eines Überdrucks und eine steuerbare Senke für Gas zur Erzeugung eines Unterdrucks. Beispielhaft umfasst das Therapiegerät 100 eine kombinierte Druckgasquelle 101 zur Erzeugung eines Unterdruckgases und eines Überdruckgases bzw. die eine Druckgasquelle 101 dient als Quelle und Senke für Gas zur steuerbaren Erzeugung eines Überdrucks und/oder Unterdrucks. Beispielsweise umfasst die Druckgasquelle 101 dazu zumindest ein Gebläse und eine Pneumatik mit Schaltmitteln 102, welche es ermöglicht, dass das Gebläse je nach Schaltung Unterdruckgas oder Überdruckgas an dem Schlauchsystem 200 bereitzustellen. Alternativ oder ergänzend können auch zwei Gebläse mit entgegengesetzter Förderrichtung vorgesehen werden, welche parallelgeschaltet sind, wobei durch Schaltmittel 102 gesteuert wird, ob Unterdruckgas oder Überdruckgas dem Schlauchsystem 200 bereitgestellt wird. Das beispielhafte Therapiegerät 100 umfasst ferner einen Speicher 103 zum Speichern von Daten, Werten und Informationen, welche beispielsweise über eine Benutzerschnittstelle 107, einen Sensor 105, einer Auswerteeinheit 106 und/oder über ein zum Datenaustausch gekoppeltes Beatmungsgerät (nicht dargestellt) eingegeben werden können. Der Speicher 103 ist dazu ausgelegt, der Steuereinheit 104, der Auswerteeinheit 106, dem Therapieerinnerungsmechanismus 108, dem Therapiewiederholungsmechanismus 109 und/oder dem Therapiestartmechanismus 110 Daten, Werte und/oder Informationen zur Verfügung zu stellen. Der Therapieerinnerungsmechanismus 108, der Therapiewiederholungsmechanismus 109 und/oder der Therapiestartmechanismus 110 können beispielsweise als Softwaremodule auf der Auswerteeinheit 106 und/oder der Steuereinheit 104 vorhanden sein. Die Therapiemechanismen 108, 109, 110 können in manchen Ausführungsformen auch eigene Hardwaremodule umfassen und/oder auf einem gemeinsamen Hardwaremodul aufgespielt sein. Auch kann vorgesehen sein, dass über die Benutzerschnittstelle 107 Daten, Werte und/oder Informationen aus dem Speicher 103 angezeigt werden können. Die im Speicher 103 abgelegten Daten, Werte und Informationen können unter anderem zumindest Parameter zur Therapie, wie etwa Therapielänge, Therapieintensität und/oder Therapieform enthalten. Auch Daten bezüglich des Patienten 300 können umfasst sein.

Beispielhaft umfasst das Therapiegerät 100 drei Therapiemechanismen: einen Therapieerinnerungsmechanismus 108, einen Therapiewiederholungsmechanismus 109 und einen Therapiestartmechanismus 110. Die einzelnen Mechanismen wurden zuvor bereits im Detail beschrieben und werden an dieser Stelle nur kurz und beispielhaft erläutert.

Der Therapieerinnerungsmechanismus 108 ist dazu eingerichtet, den Patienten 300 und/oder einen Betreuer anhand einer Datenlage an die Therapie zu erinnern. Beispielsweise dient als Datenlage zumindest eine Zeitdauer seit der letzten durchgeführten Therapie. Der Therapieerinnerungsmechanismus 108 erzeugt dazu beispielhaft einen entsprechenden Hinweis, etwa zur Anzeige auf der Benutzerschnittstelle 107. Der Hinweis kann beispielsweise darauf hinweisen, dass eine bestimmte Zeitdauer seit der letzten Therapie vergangen ist und entsprechend eine neue Therapie gestartet werden sollte. Der Hinweis kann dazu auch eine Schritt-für-Schritt-Anleitung umfassen, welche die Schritte der Therapie darstellt, etwa welche Vorbereitungen getroffen werden müssen etc. In manchen Ausführungsformen kann vorgesehen sein, dass der Patient 300 oder ein Betreuer die Erinnerung als gesehen markieren können und/oder verschieben können, sodass nach einer gewissen Zeitdauer ein neuer Hinweis generiert wird.

In manchen Ausführungsformen kann der Therapieerinnerungsmechanismus 108 auch dazu eingerichtet sein, das Therapiegerät 100 auf die Therapie vorzubereiten. Beispielsweise kann ein Therapieprogramm geladen werden, Therapieeinstellungen bzw. Therapieparameter automatisch eingestellt werden und/oder das Therapiegerät aufgewärmt/hochgefahren werden. Es kann auch vorgesehen sein, dass der Therapieerinnerungsmechanismus 108 die Therapie soweit vorbereitet, dass der Patient 300 und/oder Betreuer lediglich die Durchführung der Therapie, beispielsweise über die Benutzerschnittstelle 107, bestätigen muss. Wie bereits erläutert können neben der Zeitdauer auch andere Faktoren, Daten, Werte und/oder Informationen in die Datenlage miteinbezogen werden.

Der Therapiewiederholungsmechanismus 109 ist beispielhaft dazu eingerichtet, eine Wiederholung und/oder eine Verlängerung der Therapie anhand einer Datenlage zu starten. Die Datenlage kann dabei insbesondere Werte des Sensors 105 umfassen, welche während der Therapie erfasst werden. Beispielsweise kann der Therapiewiederholungsmechanismus 109 durch eine Auswertung der Daten darauf schließen, dass die Therapie nicht oder noch nicht ausreichend ist. Ist die Therapie nicht ausreichend, kann eine Wiederholung vorgesehen werden. Für die Wiederholung können beispielsweise die gleichen Therapieeinstellungen bzw. -parameter wieder genutzt werden. Es kann aber auch vorgesehen sein, dass bestimmte Parameter, etwa Intensität und/oder Therapieform, geändert werden um eine bessere Therapie zu erreichen. Reicht die Therapie noch nicht aus, kann beispielhaft vorgesehen sein, dass die Therapie um zumindest einen Zyklus verlängert wird, es kann auch vorgesehen sein, dass um zumindest ein oder mehrere Manöver verlängert wird. In manchen Ausführungsformen ist ein Schutzmechanismus vorgesehen, um eine zu lange Therapie zu verhindern, auch wenn diese noch nicht ausreichend ist. In dem Fall kann beispielsweise zusätzlich ein Hinweis oder ein Alarm generiert werden, dass eine maximale Therapielänge erreicht ist, diese aber noch nicht ausreichend ist.

Der Therapiestartmechanismus 110 ist beispielsweise dazu eingerichtet, die Therapie automatisch zu starten. Bezüglich der Datenlage kann der Therapiestartmechanismus 110 ähnlich wie der Therapieerinnerungsmechanismus 108 aufgebaut sein. Ein automatischer Start der Therapie ist insbesondere dann möglich, wenn das Therapiegerät 100 mit einem Beatmungsgerät 400 (nicht dargestellt) pneumatisch gekoppelt ist. Der Therapiestartmechanismus 110 kann beispielsweise dann einen automatischen Start vorsehen, wenn eine maximale Zeitdauer und/oder maximale Anzahl an Erinnerungen an die Therapie vergangen sind.

Zur Durchführung der Therapie wird die Druckgasquelle 101 und die Schaltmittel 102 entsprechend der Vorgaben, welche zum Beispiel im Speicher 103 hinterlegt sind, angesteuert. Bei der Durchführung der Therapie wird dabei dem Patienten 300 über das Schlauchsystem 200 zunächst ein Überdruckgas zur Insufflation bereitgestellt, damit der Patient 300 einatmen kann und Luft hinter das abzuhustende Sekret gelangen kann. Im Folgenden wird durch das Therapiegerät 100 bzw. durch die Schaltmittel 102 plötzlich die Bereitstellung des Überdruckgases gestoppt und/oder auf das Unterdruckgas umgeschaltet, sodass ein Hustenstoß beim Patienten 300 ausgelöst wird, durch welchen das Sekret abtransportiert werden soll. Im Zuge einer Therapie sind in der Regel mehrere Hustenstöße bzw. Zyklen vorgesehen, wobei ein Zyklus einem Einatmen (Inspiration) und einem Ausatmen (Exspiration) bzw. Husten besteht. Je nach Therapieeinstellungen kann die Intensität, etwa die Druckdifferenz und/oder die Umschaltgeschwindigkeit, der Hustenstöße variieren. Zwischen zwei Therapiezyklen ist in der Regel eine Pause zwischen 0,1 sec und 10 sec vorgesehen. In manchen Ausführungsformen kann eine Therapie auch in mehrere Manöver unterteilt sein, wobei ein Manöver wiederum mehrere Zyklen umfasst. Es kann auch vorgesehen sein, dass zwischen zwei Manövern die Pause größer ist als zwischen zwei Zyklen, beispielsweise auch mehr als 10 sec.

Nach Beendigung der Therapie kann vorgesehen sein, dass der Patient 300 und/oder ein Betreuer über die Benutzerschnittstelle 107 eine Rückmeldung zur Therapie eingeben kann, beispielsweise das subjektive Gefühl der Wirkung der Therapie. Auch kann eine weitere Durchführung der Therapie angefordert werden.

In manchen Ausführungsformen ist das System 1 oder das Therapiegerät 100 dazu ausgebildet eine Beatmung des Patienten durchzuführen. Über die Quelle für Gas zur Erzeugung eines Überdrucks kann beispielsweise eine Überdruckbeatmung bzw. eine Atmungsunterstützung des Patienten durchgeführt werden. Das Therapiegerät 100 ist in manchen Ausführungsformen also als ein Beatmungs- und Therapiegerät kombiniert. Die Steuereinheit 104 ist dabei so eingerichtet und ausgebildet, dass sie die weiteren Module, etwa die Druckgasquelle 101 und die Schaltmittel 102 so ansteuert, dass Beatmungsformen nach dem Stand der Technik durchgeführt werden können.

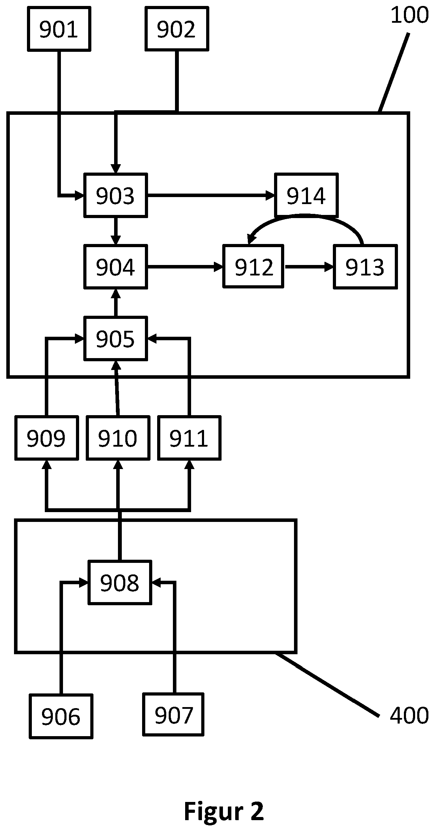

In Figur 2 ist eine schematische Darstellung der Berücksichtigung einzelner Datenströme bezüglich der Therapie dargestellt. Beispielhaft ist ein Therapiegerät 100 zumindest zum Datenaustausch mit einem Beatmungsgerät 400 gekoppelt.

Nutzereingaben 901, beispielsweise über eine Benutzerschnittstelle 107 am Therapiegerät 100 eingegeben, und Sensorwerte 902, welche beispielsweise über einen oder mehrere Sensoren 105 des Therapiegerätes 100 erfasst werden, werden beispielhaft in einer internen Verarbeitung 903 verarbeitet. Die verarbeiteten Daten können unter anderem einem Therapieerinnerungs- und/oder Therapiestartmechanismus und/oder einem Therapiewiederholungsmechanismus zur Verfügung gestellt werden. Der Startmechanismus bzw. Erinnerungsmechanismus kann beispielsweise anhand der Datenlage der internen Verarbeitung 903 einen Start/eine Erinnerung 904 an die Therapie folgen lassen, was zu einer Durchführung 912 der Therapie führt. Während der Therapie werden beispielhaft weitere Sensorwerte 902 aufgenommen, sodass über den Therapiewiederholungsmechanismus nach Ende 913 der Therapie eine Wiederholung 914 vorgesehen werden kann. Alternativ oder ergänzend kann auch anhand der Nutzereingaben 901 eine Wiederholung 914 der Therapie erfolgen.

Bei einer Kopplung des Therapiegerätes 100 mit einem Beatmungsgerät 400 können auch Daten, Werte und/oder Informationen des Beatmungsgerätes 400 für die Therapie berücksichtigt werden. Beispielsweise erfasst das Beatmungsgerät 400 eigene Nutzereingaben 906 und/oder Sensorwerte 907, welche beispielhaft in einer internen Verarbeitung 908 im Beatmungsgerät 400 aufbereitet und/oder ausgewertet werden. In manchen Ausführungsformen kann vorgesehen sein, dass das Beatmungsgerät 400 aus den Nutzereingaben 906 und/oder Sensorwerten 907 Steuerbefehle 909 ermittelt. Die optional aufbereiteten Nutzereingaben 911 und Sensorwerte 910 sowie eventuelle Steuerbefehle 909 werden an das gekoppelte Therapiegerät 100 übermittelt.

In dem Therapiegerät 100 ist beispielhaft eine Verarbeitung 905 externer Daten, wie etwa der Daten vom Beatmungsgerät 400, vorgesehen. Dabei kann vorgesehen sein, dass zumindest die Nutzereingaben 911 und/oder die Sensorwerte 910 ausgewertet werden, beispielsweise um einen Start/eine Erinnerung 904 an die Therapie zu bewirken. Beispielsweise können die Nutzereingaben 911 und/oder die Sensorwerte 910 des Beatmungsgerätes 400 darauf hindeuten, dass sich eine gewisse Menge an Sekret angesammelt hat und ein Abhusten sinnvoll wäre.

Zudem können die Steuerbefehle 909 des Beatmungsgerätes 400 an das Therapiegerät 100 durch die Verarbeitung 905 umgesetzt werden. Es kann beispielsweise vorgesehen sein, dass die interne Verarbeitung 908 der Daten im Beatmungsgerät 400 in einer Auswertung feststellt, dass eine Therapie durchgeführt werden sollte. Die Verarbeitung 905 der Steuerbefehle 909 im Therapiegerät 100 können also dazu führen, dass ein Start/eine Erinnerung 904 an die Therapie angestoßen wird.

Alternativ oder ergänzend kann das Therapiegerät 100 anhand der Nutzerdaten 901, 911 und/oder Sensordaten 902, 910 auch die Therapieeinstellungen und/oder Zeitdauer zwischen zwei Therapien bzw. bis zur nächsten Therapie anpassen

In manchen Ausführungsformen kann zudem vorgesehen sein, dass über Nutzereingaben 906 am Beatmungsgerät 400 direkt Therapieeinstellungen (Dauer, Form, Intensität, Zeitdauer zwischen zwei Therapien) vorgenommen werden können und diese an das Therapiegerät 100, beispielsweise als Steuerbefehle 909, übermittelt werden.

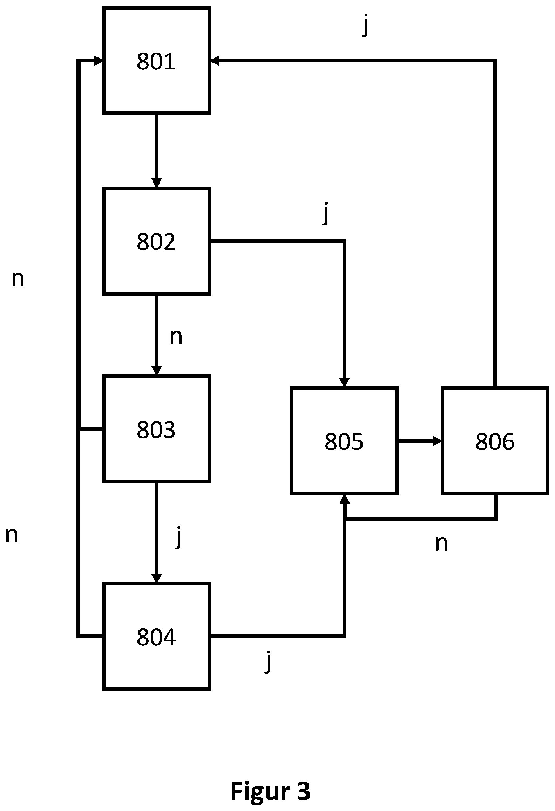

In Figur 3 ist ein stark vereinfachter Entscheidungsbaum zur Entscheidungsfindung ob ein Therapiestart, eine Therapieerinnerung und/oder eine Therapiewiederholung aktiviert werden sollte, schematisch dargestellt. In Figur 3 stellt der Buchstabe j eine positive Antwort auf die Abfrage dar, der Buchstabe n eine negative Antwort.

Ausgehend vom Startpunkt 801 ist beispielhaft die erste Abfrage 802 eine Abfrage nach der vergangenen Zeitdauer seit der letzten Therapie, ob ein gewisser Schwellwert erreicht oder übertroffen ist. Ist der Schwellwert für eine Zeitdauer seit der letzten Therapie erreicht, so wird die Abfrage 802 mit "j" gewertet. Ist die Abfrage 802 nach einem Schwellwert der Zeitdauer positiv, so folgt der Therapiestart und/oder eine Therapieerinnerung 805. Bei einer Wertung mit "n" erfolgt die nächste Abfrage.

Die nächste Abfrage 803 ist beispielhaft die Frage, ob das Therapiegerät 100 zum Datenaustausch mit einem Beatmungsgerät 400 gekoppelt ist bzw. entsprechende Daten vom Beatmungsgerät 400 übermittelt werden. Liegt keine Datenkopplung vor, erfolgt eine negative Antwort "n" und die Entscheidungsfindung beginnt wieder am Startpunkt 801. Bei einer positiven Antwort folgt die nächste Abfrage.

Beispielhaft ist die folgende Abfrage 804 eine Frage, ob die vom Beatmungsgerät 400 übermittelten Daten auf eine Ansammlung von Sekret, welches abgehustet werden sollte, hinweisen. Die Auswertung der Daten kann beispielsweise im Beatmungsgerät 400 oder durch das Therapiegerät 100 erfolgen. Bei einer negativen Antwort beginnt die Entscheidungsfindung wieder beim Startpunkt 801. Alternativ oder ergänzend können auch weitere Abfragen vorgesehen sein.

Weist die Datenlage auf eine Ansammlung von Sekret hin, so wird die Abfrage 804 positiv beantwortet und es folgt eine Therapieerinnerung und/oder ein Therapiestart 805.

Während und/oder nach der Therapie ist beispielhaft vorgesehen, dass eine Abfrage 806 nach dem Therapieergebnis, beispielsweise ob die Therapie zu ausreichenden Hustenstößen geführt hat, folgt. Ist die Therapie ausreichend gewesen beginnt die Entscheidungsfindung zum Start/zur Erinnerung der Therapie beim Startpunkt 801 erneut. Sollte die Therapie nicht ausreichend gewesen sein, kann ein erneuter Therapiestart und/oder eine Therapieerinnerung 805 vorgesehen sein.

Neben den gezeigten Abläufen kann der Entscheidungsbaum beliebig erweitert werden. Beispielsweise können weitere Daten, Werte und/oder Informationen abgefragt werden um eine Entscheidung zu treffen. Es kann beispielsweise auch vorgesehen sein, dass die Bestimmung der Zeitdauer, welche bis zur nächsten Therapie verstreichen soll anhand dieser Daten/Werte/Informationen bestimmt wird und beispielsweise zwischen Beginn 801 und der ersten Abfrage 802 nach der Zeitdauer eingefügt wird.

Liegt beispielsweise auch eine pneumatische Kopplung zwischen Therapiegerät 100 und Beatmungsgerät 400 vor, können weitere entsprechende Abfragen einfließen, etwa ob ein Wechsel des Patienteninterface nötig ist.

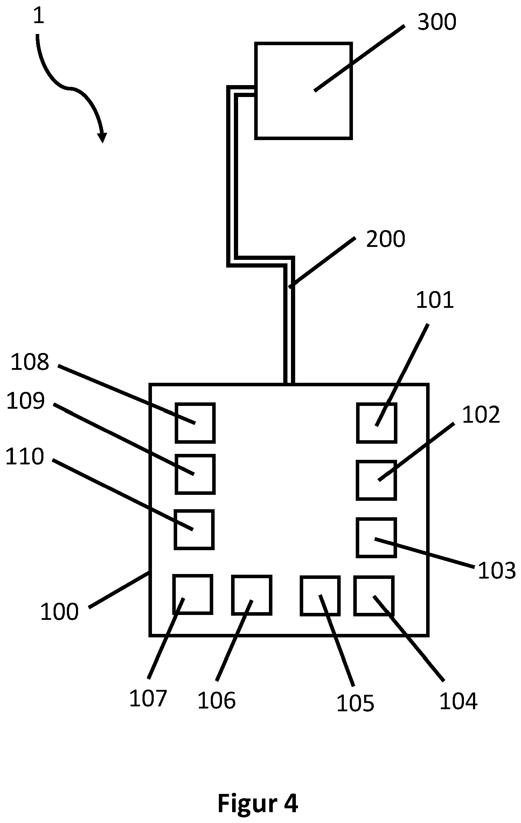

In Figur 4 ist ein beispielhaftes System 1 mit einem Therapiegerät 100 dargestellt, wobei das Therapiegerät 100 mit der steuerbaren Druckgasquelle 101 dazu eingerichtet ist zumindest eine Insufflation des über ein Schlauchsystem 200 Patienten 300 durchzuführen. Das Therapiegerät 100 ist beispielhaft dazu ausgebildet eine Therapie durchzuführen. In manchen Ausführungsformen ist das Therapiegerät 100 ein Kombinationsgerät, welches zumindest zeitweise auch als Beatmungsgerät die Beatmung des Patienten 300 leisten kann.

Das System 1 umfasst ferner Schaltmittel 102, über welche das Schlauchsystem 200 mit Überdruck beaufschlagt werden kann, beispielsweise um eine Insufflation durchzuführen. Das System 1 ist mit den Schaltmitteln 102 dazu eingerichtet über die Beaufschlagung des Schlauchsystems 200 mit Überdruck plötzlich zu stoppen und eine Exspiration und/oder ein Husten des Patienten 300 zu forcieren.

Das beispielhafte Therapiegerät 100 umfasst ferner einen Speicher 103 zum Speichern von Daten, Werten und Informationen, welche beispielsweise über eine Benutzerschnittstelle 107, einen Sensor 105, einer Auswerteeinheit 106 und/oder über ein zum Datenaustausch gekoppeltes Beatmungsgerät (nicht dargestellt) eingegeben werden können. Der Speicher 103 ist dazu ausgelegt, der Steuereinheit 104, der Auswerteeinheit 106, dem Therapieerinnerungsmechanismus 108, dem Therapiewiederholungsmechanismus 109 und/oder dem Therapiestartmechanismus 110 Daten, Werte und/oder Informationen zur Verfügung zu stellen. Der Therapieerinnerungsmechanismus 108, der Therapiewiederholungsmechanismus 109 und/oder der Therapiestartmechanismus 110 können beispielsweise als Softwaremodule auf der Auswerteeinheit 106 und/oder der Steuereinheit 104 vorhanden sein. Die Therapiemechanismen 108, 109, 110 können in manchen Ausführungsformen auch eigene Hardwaremodule umfassen und/oder auf einem gemeinsamen Hardwaremodul aufgespielt sein. Auch kann vorgesehen sein, dass über die Benutzerschnittstelle 107 Daten, Werte und/oder Informationen aus dem Speicher 103 angezeigt werden können. Die im Speicher 103 abgelegten Daten, Werte und Informationen können unter anderem zumindest Parameter zur Therapie, wie etwa Therapielänge, Therapieintensität und/oder Therapieform enthalten. Auch Daten bezüglich des Patienten 300 können umfasst sein.

Beispielhaft umfasst das Therapiegerät 100 drei Therapiemechanismen: einen Therapieerinnerungsmechanismus 108, einen Therapiewiederholungsmechanismus 109 und einen Therapiestartmechanismus 110. Die einzelnen Mechanismen wurden zuvor bereits im Detail beschrieben und werden an dieser Stelle nur kurz und beispielhaft erläutert.

Der Therapieerinnerungsmechanismus 108 ist dazu eingerichtet, den Patienten 300 und/oder einen Betreuer anhand einer Datenlage an die Therapie zu erinnern. Beispielsweise dient als Datenlage zumindest eine Zeitdauer seit der letzten durchgeführten Therapie. Der Therapieerinnerungsmechanismus 108 erzeugt dazu beispielhaft einen entsprechenden Hinweis, etwa zur Anzeige auf der Benutzerschnittstelle 107. Der Hinweis kann beispielsweise darauf hinweisen, dass eine bestimmte Zeitdauer seit der letzten Therapie vergangen ist und entsprechend eine neue Therapie gestartet werden sollte. Der Hinweis kann dazu auch eine Schritt-für-Schritt-Anleitung umfassen, welche die Schritte der Therapie darstellt, etwa welche Vorbereitungen getroffen werden müssen etc. In manchen Ausführungsformen kann vorgesehen sein, dass der Patient 300 oder ein Betreuer die Erinnerung als gesehen markieren können und/oder verschieben können, sodass nach einer gewissen Zeitdauer ein neuer Hinweis generiert wird.

In manchen Ausführungsformen kann der Therapieerinnerungsmechanismus 108 auch dazu eingerichtet sein, das Therapiegerät 100 auf die Therapie vorzubereiten. Beispielsweise kann ein Therapieprogramm geladen werden, Therapieeinstellungen bzw. Therapieparameter automatisch eingestellt werden und/oder das Therapiegerät aufgewärmt/hochgefahren werden. Es kann auch vorgesehen sein, dass der Therapieerinnerungsmechanismus 108 die Therapie soweit vorbereitet, dass der Patient 300 und/oder Betreuer lediglich die Durchführung der Therapie, beispielsweise über die Benutzerschnittstelle 107, bestätigen muss. Wie bereits erläutert können neben der Zeitdauer auch andere Faktoren, Daten, Werte und/oder Informationen in die Datenlage miteinbezogen werden.

Der Therapiewiederholungsmechanismus 109 ist beispielhaft dazu eingerichtet, eine Wiederholung und/oder eine Verlängerung der Therapie anhand einer Datenlage zu starten. Die Datenlage kann dabei insbesondere Werte des Sensors 105 umfassen, welche während der Therapie erfasst werden. Beispielsweise kann der Therapiewiederholungsmechanismus 109 durch eine Auswertung der Daten darauf schließen, dass die Therapie nicht oder noch nicht ausreichend ist. Ist die Therapie nicht ausreichend, kann eine Wiederholung vorgesehen werden. Für die Wiederholung können beispielsweise die gleichen Therapieeinstellungen bzw. -parameter wieder genutzt werden. Es kann aber auch vorgesehen sein, dass bestimmte Parameter, etwa Intensität und/oder Therapieform, geändert werden um eine bessere Therapie zu erreichen. Reicht die Therapie noch nicht aus, kann beispielhaft vorgesehen sein, dass die Therapie um zumindest einen Zyklus verlängert wird, es kann auch vorgesehen sein, dass um zumindest ein oder mehrere Manöver verlängert wird. In manchen Ausführungsformen ist ein Schutzmechanismus vorgesehen, um eine zu lange Therapie zu verhindern, auch wenn diese noch nicht ausreichend ist. In dem Fall kann beispielsweise zusätzlich ein Hinweis oder ein Alarm generiert werden, dass eine maximale Therapielänge erreicht ist, diese aber noch nicht ausreichend ist.

Der Therapiestartmechanismus 110 ist beispielsweise dazu eingerichtet, die Therapie automatisch zu starten. Bezüglich der Datenlage kann der Therapiestartmechanismus 110 ähnlich wie der Therapieerinnerungsmechanismus 108 aufgebaut sein. Ein automatischer Start der Therapie ist insbesondere dann möglich, wenn das Therapiegerät 100 mit einem Beatmungsgerät 400 (nicht dargestellt) pneumatisch gekoppelt ist. Der Therapiestartmechanismus 110 kann beispielsweise dann einen automatischen Start vorsehen, wenn eine maximale Zeitdauer und/oder maximale Anzahl an Erinnerungen an die Therapie vergangen sind.

Zur Durchführung der Therapie wird die Druckgasquelle 101 und die Schaltmittel 102 entsprechend der Vorgaben, welche zum Beispiel im Speicher 103 hinterlegt sind, angesteuert. Bei der Durchführung der Therapie wird dabei dem Patienten 300 über das Schlauchsystem 200 zunächst ein Überdruckgas zur Insufflation bereitgestellt, damit der Patient 300 einatmen kann und Luft hinter das abzuhustende Sekret gelangen kann. Im Folgenden wird durch das Therapiegerät 100 bzw. durch die Schaltmittel 102 plötzlich die Bereitstellung des Überdruckgases gestoppt sodass ein Hustenstoß beim Patienten 300 ausgelöst wird, durch welchen das Sekret abtransportiert werden soll. Im Zuge einer Therapie sind in der Regel mehrere Hustenstöße bzw. Zyklen vorgesehen, wobei ein Zyklus einem Einatmen (Inspiration) und einem Ausatmen (Exspiration) bzw. Husten besteht. Je nach Therapieeinstellungen kann die Intensität, etwa die Druckdifferenz und/oder die Umschaltgeschwindigkeit, der Hustenstöße variieren. Zwischen zwei Therapiezyklen ist in der Regel eine Pause zwischen 0,1 sec und 10 sec vorgesehen. In manchen Ausführungsformen kann eine Therapie auch in mehrere Manöver unterteilt sein, wobei ein Manöver wiederum mehrere Zyklen umfasst. Es kann auch vorgesehen sein, dass zwischen zwei Manövern die Pause größer ist als zwischen zwei Zyklen, beispielsweise auch mehr als 10 sec.

Nach Beendigung der Therapie kann vorgesehen sein, dass der Patient 300 und/oder ein Betreuer über die Benutzerschnittstelle 107 eine Rückmeldung zur Therapie eingeben kann, beispielsweise das subjektive Gefühl der Wirkung der Therapie. Auch kann eine weitere Durchführung der Therapie angefordert werden.

In manchen Ausführungsformen ist das System 1 oder das Therapiegerät 100 dazu ausgebildet eine Beatmung des Patienten durchzuführen. Über die Quelle für Gas zur Erzeugung eines Überdrucks kann beispielsweise eine Überdruckbeatmung bzw. eine Atmungsunterstützung des Patienten durchgeführt werden. Das Therapiegerät 100 ist in manchen Ausführungsformen also als ein Beatmungs- und Therapiegerät kombiniert. Die Steuereinheit 104 ist dabei so eingerichtet und ausgebildet, dass sie die weiteren Module, etwa die Druckgasquelle 101 und die Schaltmittel 102 so ansteuert, dass Beatmungsformen nach dem Stand der Technik durchgeführt werden können.

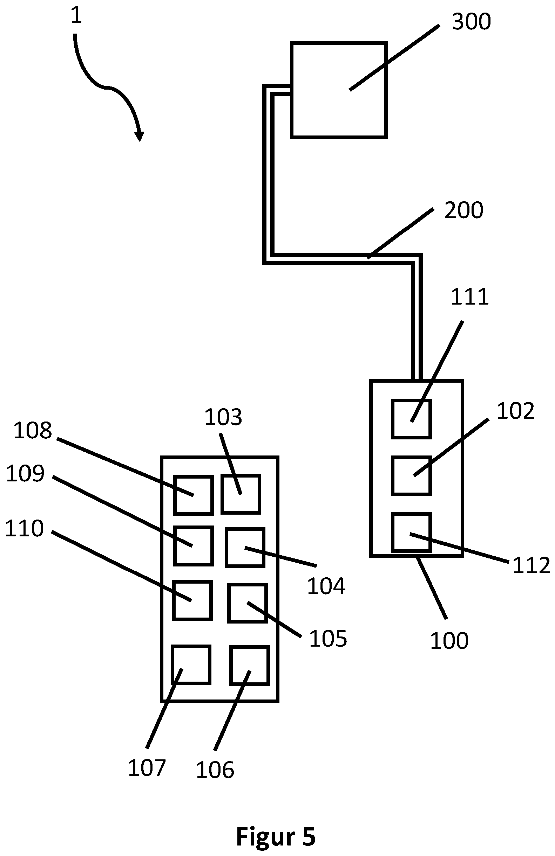

Figur 5 zeigt ein beispielhaftes System 1 mit einem Therapiegerät 100, wobei das Therapiegerät 100 eine Senke für Gas zur Erzeugung eines Unterdrucks im Schlauchsystem 200 umfasst. Über den Unterdruck ist es beispielsweise möglich Sekret aus dem Mund- und/oder Rachenraum des Patienten 300 abzusaugen.

In dem beispielhaft dargestellten System 1 ist die Steuereinheit 104 mit den Mechanismen 108, 109, 110 sowie der Speicher 103, der Sensor 105, die Auswerteeinheit 106 und die Benutzerschnittstelle 107 zumindest räumlich unabhängig vom Therapiegerät 100 angeordnet. In manchen Ausführungsformen können die Steuereinheit 104 mit den Mechanismen 108, 109, 110 sowie der Speicher 103, der Sensor 105, die Auswerteeinheit 106 und die Benutzerschnittstelle 107 in einem Beatmungsgerät angeordnet sein oder als separates Gerät zusammengefasst sein, welches die Atmung und/oder zumindest die Sekretsituation des Patienten überwacht.

Das beispielhafte System 100 umfasst einen Speicher 103 zum Speichern von Daten, Werten und Informationen, welche beispielsweise über eine Benutzerschnittstelle 107, einen Sensor 105, einer Auswerteeinheit 106 und/oder über ein zum Datenaustausch gekoppeltes Beatmungsgerät (nicht dargestellt) eingegeben werden können. Der Speicher 103 ist dazu ausgelegt, der Steuereinheit 104, der Auswerteeinheit 106, dem Therapieerinnerungsmechanismus 108, dem Therapiewiederholungsmechanismus 109 und/oder dem Therapiestartmechanismus 110 Daten, Werte und/oder Informationen zur Verfügung zu stellen. Der Therapieerinnerungsmechanismus 108, der Therapiewiederholungsmechanismus 109 und/oder der Therapiestartmechanismus 110 können beispielsweise als Softwaremodule auf der Auswerteeinheit 106 und/oder der Steuereinheit 104 vorhanden sein. Die Therapiemechanismen 108, 109, 110 können in manchen Ausführungsformen auch eigene Hardwaremodule umfassen und/oder auf einem gemeinsamen Hardwaremodul aufgespielt sein. Auch kann vorgesehen sein, dass über die Benutzerschnittstelle 107 Daten, Werte und/oder Informationen aus dem Speicher 103 angezeigt werden können. Die im Speicher 103 abgelegten Daten, Werte und Informationen können unter anderem zumindest Parameter zur Therapie, wie etwa Therapielänge, Therapieintensität und/oder Therapieform enthalten. Auch Daten bezüglich des Patienten 300 können umfasst sein.

Beispielhaft umfasst das System 100 drei Therapiemechanismen: einen Therapieerinnerungsmechanismus 108, einen Therapiewiederholungsmechanismus 109 und einen Therapiestartmechanismus 110. Die einzelnen Mechanismen wurden zuvor bereits im Detail beschrieben und werden an dieser Stelle nur kurz und beispielhaft erläutert.

Der Therapieerinnerungsmechanismus 108 ist dazu eingerichtet, den Patienten 300 und/oder einen Betreuer anhand einer Datenlage an die Therapie zu erinnern. Beispielsweise dient als Datenlage zumindest eine Zeitdauer seit der letzten durchgeführten Therapie. Der Therapieerinnerungsmechanismus 108 erzeugt dazu beispielhaft einen entsprechenden Hinweis, etwa zur Anzeige auf der Benutzerschnittstelle 107. Der Hinweis kann beispielsweise darauf hinweisen, dass eine bestimmte Zeitdauer seit der letzten Therapie vergangen ist und entsprechend eine neue Therapie gestartet werden sollte. Der Hinweis kann dazu auch eine Schritt-für-Schritt-Anleitung umfassen, welche die Schritte der Therapie darstellt, etwa welche Vorbereitungen getroffen werden müssen etc. In manchen Ausführungsformen kann vorgesehen sein, dass der Patient 300 oder ein Betreuer die Erinnerung als gesehen markieren können und/oder verschieben können, sodass nach einer gewissen Zeitdauer ein neuer Hinweis generiert wird.

In manchen Ausführungsformen kann der Therapieerinnerungsmechanismus 108 auch dazu eingerichtet sein, das Therapiegerät 100 auf die Therapie vorzubereiten. Beispielsweise kann ein Therapieprogramm geladen werden, Therapieeinstellungen bzw. Therapieparameter automatisch eingestellt werden und/oder das Therapiegerät aufgewärmt/hochgefahren werden. Es kann auch vorgesehen sein, dass der Therapieerinnerungsmechanismus 108 die Therapie soweit vorbereitet, dass der Patient 300 und/oder Betreuer lediglich die Durchführung der Therapie, beispielsweise über die Benutzerschnittstelle 107, bestätigen muss. Wie bereits erläutert können neben der Zeitdauer auch andere Faktoren, Daten, Werte und/oder Informationen in die Datenlage miteinbezogen werden.

Der Therapiewiederholungsmechanismus 109 ist beispielhaft dazu eingerichtet, eine Wiederholung und/oder eine Verlängerung der Therapie anhand einer Datenlage zu starten. Die Datenlage kann dabei insbesondere Werte des Sensors 105 umfassen, welche während der Therapie erfasst werden. Beispielsweise kann der Therapiewiederholungsmechanismus 109 durch eine Auswertung der Daten darauf schließen, dass die Therapie nicht oder noch nicht ausreichend ist. Ist die Therapie nicht ausreichend, kann eine Wiederholung vorgesehen werden. Für die Wiederholung können beispielsweise die gleichen Therapieeinstellungen bzw. -parameter wieder genutzt werden. Es kann aber auch vorgesehen sein, dass bestimmte Parameter, etwa Intensität und/oder Therapieform, geändert werden um eine bessere Therapie zu erreichen. Reicht die Therapie noch nicht aus, kann beispielhaft vorgesehen sein, dass die Therapie um zumindest eine Durchführung verlängert wird. In manchen Ausführungsformen ist ein Schutzmechanismus vorgesehen, um eine zu lange Therapie zu verhindern, auch wenn diese noch nicht ausreichend ist. In dem Fall kann beispielsweise zusätzlich ein Hinweis oder ein Alarm generiert werden, dass eine maximale Therapielänge erreicht ist, diese aber noch nicht ausreichend ist.

Der Therapiestartmechanismus 110 ist beispielsweise dazu eingerichtet, die Therapie automatisch zu starten. Bezüglich der Datenlage kann der Therapiestartmechanismus 110 ähnlich wie der Therapieerinnerungsmechanismus 108 aufgebaut sein. Ein automatischer Start der Therapie ist insbesondere dann möglich, wenn das Therapiegerät 100 mit einem Beatmungsgerät pneumatisch gekoppelt ist oder das Therapiegerät 100 in ein Beatmungsgerät integriert ist. Der Therapiestartmechanismus 110 kann beispielsweise dann einen automatischen Start vorsehen, wenn eine maximale Zeitdauer und/oder maximale Anzahl an Erinnerungen an die Therapie vergangen sind.

Zur Durchführung der Therapie wird durch die Senke 111 ein Unterdruck am Schlauchsystem 200 erzeugt, wobei der Unterdruck durch das Schaltmittel 102 dem Schlauchsystem 200 bereitgestellt werden kann und auch die Bereitstellung gestoppt werde kann. Das Schlauchsystem 200 ist beispielsweise ein Schlauch, optional mit einem speziellen Endstück zum Absaugen von Sekret. Über den Schlauch wird das Sekret vom Patienten abgesaugt und ggf. in einem Auffangbehälter 112 im bzw. am Therapiegerät 100 aufgefangen.

Nach Beendigung der Therapie kann vorgesehen sein, dass der Patient 300 und/oder ein Betreuer über die Benutzerschnittstelle 107 eine Rückmeldung zur Therapie eingeben kann, beispielsweise das subjektive Gefühl der Wirkung der Therapie. Auch kann eine weitere Durchführung der Therapie angefordert werden.

In manchen Ausführungsformen kann das Therapiegerät 100 im System 1 auch mit einem Beatmungsgerät kombiniert und/oder gekoppelt werden. Beispielsweise kann das Beatmungsgerät dazu eingerichtet sein eine Beatmung des Patienten über eine Trachealkanüle durchzuführen. Das Therapiegerät 100 kann dabei so mit dem Beatmungsgerät kombiniert und/oder in das Beatmungsgerät integriert sein, dass ein Absaugen von Sekret über beispielsweise die Trachealkanüle möglich ist.

### Bezugszeichenliste

1 System
100 Therapiegerät
101 Druckgasquelle
102 Schaltmittel
103 Speicher
104 Steuereinheit
105 Sensor
106 Auswerteeinheit
107 Benutzerschnittstelle
108 Therapieerinnerungsmechanismus
109 Therapiewiederholungsmechanismus
110 Therapiestartmechanismus
111 Senke
112 Auffangbehälter
801 Startpunkt
802 Abfrage nach vergangener Zeitdauer
803 Abfrage nach Datenaustausch
804 Abfrage nach Sekretansammlung
805 Start/Erinnerung Therapie
806 Abfrage nach Therapieergebnis
901 Nutzereingaben
902 Sensorwerte
903 Interne Verarbeitung
904 Start-/Erinnerungsmechanismus
905 Verarbeitung externer Daten
906 Nutzereingaben
907 Sensorwerte
908 Interne Verarbeitung
909 Steuerbefehle
910 Sensorwerte
911 Nutzereingaben
912 Durchführung der Therapie
913 Ende der Therapie
914 Wiederholungsmechanismus

## Patentansprüche

1. System (1) mit zumindest einem Therapiegerät (100) zur Durchführung einer Therapie und mindestens einem Schlauchsystem (200) zur gasleitenden Verbindung zwischen dem Therapiegerät (100) und einem Patienten (300),
wobei das Therapiegerät (100) zumindest eine Quelle (101) für Gas zur Erzeugung eines Überdrucks und/oder zumindest eine Senke (101) für Gas zur Erzeugung eines Unterdrucks aufweist und ausgebildet ist, um dem Schlauchsystem (200) zumindest zeitweise einen Überdruck und/oder Unterdruck zur Durchführung der Therapie bereitzustellen,
wobei das System (1) ferner zumindest eine Steuereinheit (104), zumindest eine Benutzerschnittstelle (107), zumindest einen Speicher (103) und zumindest einen Sensor (105) umfasst,
wobei die Steuereinheit (104) mindestens einen der folgenden Therapiemechanismen aufweist: einen Therapiestartmechanismus (110) zum Starten der Therapie, einen Therapieerinnerungsmechanismus (108) zur Erinnerung an die Therapie, einen Therapiewiederholungsmechanismus zur Verlängerung und/oder Wiederholung der Therapie, wobei jeder der Therapiemechanismen (108, 109, 110) auf Nutzeraktionen, Patienten- und/oder Therapiedaten basiert,
wobei das Therapiegerät (100) mit einem Beatmungsgerät zur Datenübermittlung gekoppelt ist, wobei das Beatmungsgerät ausgebildet ist, um Daten für den mindestens einen Therapiemechanismus bereitzustellen,
wobei jeder der Therapiemechanismen (108, 109, 110) ferner auf einer Datenlage basiert, die vom Beatmungsgerät während einer Beatmung erfasste Sensordaten und Werte als Beatmungsdaten umfasst, wobei das System (1) ausgebildet ist die erfassten Sensordaten, die auf eine zunehmende Verschlechterung und/oder Verbesserung einer Ansammlung von Sekret beim Patienten hinweisen, zu ermitteln und abhängig von der Verschlechterung und/oder Verbesserung automatisch eine Therapieintensität anzupassen,
wobei das System (1) ausgebildet ist, um zur Durchführung der Therapie eine Druckgasquelle (101) und Schaltmittel (102) so anzusteuern, dass mehrere Hustenstöße beim Patienten (300) ausgelöst werden, wobei zum Auslösen eines Hustenstoßes dem Patienten (300) über das Schlauchsystem (200) ein Überdruckgas zur Insufflation bereitgestellt wird und anschließend durch die Schaltmittel (102) die Bereitstellung des Überdruckgases plötzlich gestoppt und/oder auf ein Unterdruckgas umgeschaltet wird, wobei eine Intensität der Hustenstöße je nach Therapieeinstellungen durch Variieren einer Druckdifferenz und/oder einer Umschaltgeschwindigkeit variiert wird.

2. System nach Anspruch 1,
wobei die Therapie eine Hustentherapie, eine Hustenunterstützung, eine Insufflation, eine Insufflation mit nachfolgender Exsufflation, ein Absaugen von Sekret aus dem Mund oder den Atemwegen oder eine Kombination von mindestens zwei dieser Beispiele umfasst.

3. System nach einem der vorhergehenden Ansprüche,
wobei die Steuereinheit ausgebildet ist, um über die Benutzerschnittstelle und/oder über eine zusätzliche Schnittstelle und/oder aus dem Speicher den Patienten charakterisierende Daten, insbesondere bezüglich einer Lungenfunktion, eines Alters, eines Gewichts, einer Beatmung, einer Grunderkrankung, einer Vorerfahrung mit Hustenmanövern, für eine Häufigkeit der Erinnerung an die Therapie und/oder des Starts der Therapie automatisch anzupassen.

4. System nach einem der vorhergehenden Ansprüche,
wobei das System ausgebildet ist, um auf Basis von ermittelten Daten, insbesondere von Messwerten und gespeicherten Werten zu SpO2, Puls, CO2 und/oder einem sich verändernden Druck- und/oder Volumenflussverhalten, die Therapie automatisch zu starten, dem Patienten den Start der Therapie anzubieten oder an die Therapie zu erinnern, wenn die ermittelten Daten anzeigen, dass eine Sekretentfernung aus den Atemwegen durch eine Therapie förderlich wäre.

5. System nach einem der vorhergehenden Ansprüche,
wobei das System ausgebildet ist, um Beatmungsparameter auf dem Beatmungsgerät abhängig von einer ermittelten Auswirkung durchgeführter Therapien auf einen Verlauf von mindestens einer der folgenden Größen zu verändern: SpO2, CO2, Fluss, Tidalvolumen, Signal-Rausch-Verhältnis (SNR).

6. System nach einem der vorhergehenden Ansprüche,
wobei das Therapiegerät und das Beatmungsgerät pneumatisch koppelbar sind, sodass eine Beatmung und eine maschinelle Insufflation und Exsufflation direkt kombiniert werden können.

7. System nach einem der vorhergehenden Ansprüche,
wobei das System so ausgebildet ist, dass die Erinnerung an die Therapie oder der Start der Therapie ausschließlich oder zusätzlich über das gekoppelte Beatmungsgerät erfolgt.

8. System nach einem der vorhergehenden Ansprüche,
wobei die Daten, auf denen der mindestens eine Therapiemechanismus basiert, einen SpO2-Wert umfassen, wobei das System ausgebildet ist, um mindestens eine der folgenden Funktionen auszuführen, wenn der SpO2-Wert einen SpO2-Schwellenwert erreicht: Erinnerung an die Therapie, Start der Therapie, Wiederholung der Therapie, Verlängerung der Therapie.

9. System nach einem der vorhergehenden Ansprüche,
wobei das System ausgebildet ist, um eine Wirkung der durchgeführten Therapie auf einen Verlauf von mindestens einer der folgenden Größen zu speichern und/oder zu verarbeiten: SpO2, CO2, Druck, Fluss, Tidalvolumen, akustische Signale, Signal-Rausch-Verhältnis (SNR) im Therapiegerät und/oder in einem gekoppelten Beatmungsgerät;
wobei das System ferner ausgebildet ist, um die gespeicherten und/oder verarbeiteten Daten bei Entscheidungen zu mindestens einer der folgenden Funktionen zu berücksichtigen: Start der Therapie, Wiederholung der Therapie, Erinnerung an die Therapie.

10. System nach einem der vorhergehenden Ansprüche,
wobei das System ausgebildet ist, um das Signal-Rausch-Verhältnis (SNR) von Druck- und/oder Flusssignalen zu evaluieren und einen Start der Therapie oder eine Erinnerung an die Therapie einzuleiten oder zu verlängern, wenn der SNR-Wert einen SNR-Schwellenwert erreicht.

11. System nach einem der vorhergehenden Ansprüche,
wobei das System ausgebildet ist, um auf Basis von ermittelten Daten, insbesondere von Messwerten und gespeicherten Werten zu Druck, Fluss, Tidalvolumen, SpO2, Puls und/oder CO2, oder auf Basis von Daten aus vergangenen Hustenmanövern, insbesondere einem Hustenfluss, Wiederholungen von In- und Exsufflationen automatisch durchzuführen und/oder dem Patienten anzubieten, wenn diese Daten anzeigen, dass weiteres Sekret aus den Atemwegen entfernt werden sollte.

12. System nach einem der vorhergehenden Ansprüche,
wobei das System ausgebildet ist, um ermittelte und/oder gespeicherte Daten in ein cloudbasiertes System hochzuladen, therapiebezogene Entscheidungen mittels cloudbasierter Algorithmen zu treffen und die Entscheidungen dem Therapiegerät wiederum aus der Cloud für die Anpassung der Therapie oder von Startzeitpunkten der Therapie zur Verfügung zu stellen.

13. System nach einem der vorhergehenden Ansprüche,
wobei das System ausgebildet ist, um eine Hustentherapie infolge der Erinnerung an die Therapie durch das Therapiegerät über eine Fernsteuerung und/oder eine App auf einem mit dem Therapiegerät gekoppelten mobilen Endgerät anwenderseitig zu starten und/oder zu wiederholen und/oder zu beenden.

14. System nach einem der vorhergehenden Ansprüche,
wobei das System ausgebildet ist, um die Therapie auf Basis von ermittelten Daten automatisch zu starten, wobei die Therapieparameter anhand der ermittelten Daten automatisch angepasst werden.

15. System nach einem der vorhergehenden Ansprüche,
wobei die zumindest eine Steuereinheit, der zumindest eine Speicher, die zumindest eine Benutzerschnittstelle und der zumindest eine Sensor Teil des Therapiegerätes sind.

## Claims

1. A system (1) comprising at least one therapy device (100) for performing a therapy and at least one hose system (200) for gas-conducting connection between the therapy device (100) and a patient (300),
wherein the therapy device (100) has at least one source (101) for gas for generating an overpressure and/or at least one sink (101) for gas for generating a negative pressure and is designed to provide, at least temporarily, an overpressure and/or negative pressure to the hose system (200) in order to perform the therapy,
wherein the system (1) also comprises at least one control unit (104), at least one user interface (107), at least one memory (103), and at least one sensor (105),
wherein the control unit (104) has at least one of the following therapy mechanisms: a therapy start mechanism (110) for starting the therapy, a therapy reminder mechanism (108) for reminding about the therapy, a therapy repetition mechanism for extending and/or repeating the therapy, wherein each of the therapy mechanisms (108, 109, 110) is based on user actions, patient data, and/or therapy data,
wherein the therapy device (100) is coupled to a ventilator for data transmission, wherein the ventilator is designed to provide data for the at least one therapy mechanism,
wherein each of the therapy mechanisms (108, 109, 110) is also based on an availability of data which comprises sensor data and values recorded by the ventilator during ventilation as ventilation data, wherein the system is designed to determine the recorded sensor data that indicate an increasing worsening and/or improvement of an accumulation of secretion in the patient and to automatically adapt a therapy intensity depending on the worsening and/or improvement,
wherein the system (1) is designed, in order to perform the therapy, to actuate a pressurized gas source (101) and switching means (102) such that multiple coughs are triggered in the patient (300), wherein, in order to trigger a cough in the patient (300), an overpressure gas for insufflation is provided via the hose system (200) and then, by means of the switching means (102), the provision of the overpressure gas is suddenly stopped and/or switched to a negative pressure gas, wherein an intensity of the coughs is varied depending on the therapy settings by varying a pressure difference and/or a switching speed.

2. The system according to claim 1,
wherein the therapy comprises cough therapy, cough support, insufflation, insufflation with subsequent exsufflation, aspirating secretion from the mouth or the airways, or a combination of at least two of these examples.

3. The system according to any one of the preceding claims,
wherein the control unit is designed to automatically adapt, via the user interface and/or via an additional interface and/or from the memory, data characterizing the patient, in particular with regard to lung function, age, weight, ventilation, underlying illness, prior experience with cough maneuvers, for a frequency of the reminder about the therapy and/or of the start of the therapy.

4. The system according to any one of the preceding claims,
wherein the system is designed, on the basis of determined data, in particular measured values and stored values for SpO2, pulse, CO2, and/or a variable pressure and/or volume flow behavior, to automatically start the therapy, to offer to the patient to start the therapy, or to remind the patient about the therapy when the determined data show that secretion removal from the airways by means of a therapy would be beneficial.

5. The system according to any one of the preceding claims,
wherein the system is designed to modify ventilation parameters at the ventilator depending on a determined impact of performed therapies on a progression of at least one of the following variables: SpO2, CO2, flow, tidal volume, signal-to-noise ratio (SNR).

6. The system according to any one of the preceding claims,
wherein the therapy device and the ventilator can be pneumatically coupled such that ventilation and machine insufflation and exsufflation can be directly combined.

7. The system according to any one of the preceding claims,
wherein the system is designed such that the reminder about the therapy or the start of the therapy take place exclusively or additionally via the coupled ventilator.

8. The system according to any one of the preceding claims,
wherein the data on which the at least one therapy mechanism is based comprises an SpO2 value, wherein the system is designed to carry out at least one of the following functions when the SpO2 value reaches an SpO2 threshold: reminder about the therapy, start of the therapy, repetition of the therapy, extension of the therapy.

9. The system according to any one of the preceding claims,
wherein the system is designed to store and/or process an effect of the performed therapy on a progression of at least one of the following variables: SpO2, CO2, pressure, flow, tidal volume, acoustic signals, signal-to-noise ratio (SNR) in the therapy device and/or in a coupled ventilator;
wherein the system is also designed to take into account the stored and/or processed data in the case of decisions regarding at least one of the following functions: start of the therapy, repetition of the therapy, reminder about the therapy.

10. The system according to any one of the preceding claims,
wherein the system is designed to evaluate the signal-to-noise ratio (SNR) of pressure and/or flow signals and to initiate or extend a start of the therapy or a reminder about the therapy when the SNR value reaches an SNR threshold.

11. The system according to any one of the preceding claims,
wherein the system is designed, on the basis of determined data, in particular measured values and stored values regarding pressure, flow, tidal volume, SpO2, pulse, and/or CO2, or on the basis of data from previous cough maneuvers, in particular a cough flow, to automatically perform and/or offer the patient repetitions of insufflation and exsufflation when this data shows that further secretion should be removed from the airways.

12. The system according to any one of the preceding claims,
wherein the system is designed to upload determined and/or stored data to a cloud-based system, make therapy-related decisions by means of cloud-based algorithms, and in turn to provide the decisions to the therapy device from the cloud in order to adapt the therapy or start times of the therapy.

13. The system according to any one of the preceding claims,
wherein the system is designed to start and/or repeat and/or terminate cough therapy from the user's side as a result of the reminder about the therapy by the therapy device via a remote control and/or an app on a mobile device coupled to the therapy device.

14. The system according to any one of the preceding claims,
wherein the system is designed to automatically start the therapy on the basis of determined data, wherein the therapy parameters are automatically adapted using the determined data.

15. The system according to any one of the preceding claims,
wherein the at least one control unit, the at least one memory, the at least one user interface, and the at least one sensor are part of the therapy device.

## Revendications

1. Système (1) comprenant au moins un appareil de thérapie (100) pour la mise en œuvre d'une thérapie et au moins un système de tuyaux (200) pour une liaison conductrice de gaz entre l'appareil de thérapie (100) et un patient (300),
dans lequel l'appareil de thérapie (100) présente au moins une source de gaz (101) destinée à produire une surpression et/ou au moins un puits de gaz (101) destiné à produire une dépression et est conçu pour fournir au moins temporairement une surpression et/ou une dépression au système de tuyaux (200) pour la mise en œuvre de la thérapie,
dans lequel le système (1) comporte en outre au moins une unité de commande (104), au moins une interface d'utilisateur (107), au moins une mémoire (103) et au moins un capteur (105),
dans lequel l'unité de commande (104) présente au moins l'un des mécanismes de thérapie suivants : un mécanisme de démarrage de thérapie (110) pour le démarrage de la thérapie, un mécanisme de rappel de thérapie (108) pour le rappel de la thérapie, un mécanisme de répétition de thérapie pour le prolongement et/ou la répétition de la thérapie, dans lequel chacun des mécanismes de thérapie (108, 109, 110) est basé sur des actions d'utilisateur, des données de patient et/ou de thérapie,
dans lequel l'appareil de thérapie (100) est couplé à un appareil respiratoire pour la transmission de données, dans lequel l'appareil respiratoire est conçu pour fournir des données pour l'au moins un mécanisme de thérapie,
dans lequel chacun des mécanismes de thérapie (108, 109, 110) est en outre basé sur une base de données comportant des valeurs et des données de capteur détectées par l'appareil respiratoire pendant une ventilation en tant que données de ventilation, dans lequel le système est conçu pour déterminer les données de capteur détectées indiquant une détérioration et/ou une amélioration croissante d'un accumulation de sécrétions chez le patient, et pour adapter automatiquement une intensité de la thérapie en fonction de la détérioration et/ou de l'amélioration, dans lequel, pour la mise en œuvre de la thérapie, le système (1) est conçu pour activer une source de gaz sous pression (101) et des moyens de commutation (102) de manière à déclencher plusieurs quintes de toux chez le patient (300), dans lequel un gaz en surpression est fourni au patient (300) pour l'insufflation par le biais du système de tuyaux (200) afin de déclencher une quinte de toux et l'apport du gaz en surpression est ensuite arrêté soudainement et/ou commuté sur un gaz en dépression par les moyens de commutation (102), dans lequel une intensité des quintes de toux est modifiée en fonction des réglages de la thérapie par modification d'une différence de pression et/ou d'une vitesse de commutation.

2. Système selon la revendication 1,
dans lequel la thérapie comporte un traitement de la toux, une assistance à la toux, une insufflation, une insufflation suivie d'une exsufflation, une aspiration de sécrétions à partir de la bouche ou des voies respiratoires ou une combinaison d'au moins deux de ces exemples.

3. Système selon l'une des revendications précédentes,
dans lequel l'unité de commande est conçue pour adapter automatiquement des données caractérisant le patient, concernant en particulier une fonction pulmonaire, un âge, un poids, une ventilation, une pathologie sous-jacente, une expérience préalable en matière d'exercices pour la toux, par le biais de l'interface d'utilisateur et/ou par le biais d'une interface supplémentaire et/ou à partir de la mémoire, pour une fréquence du rappel de la thérapie et/ou du démarrage de la thérapie.

4. Système selon l'une des revendications précédentes,
dans lequel le système est conçu pour démarrer automatiquement la thérapie, proposer le démarrage de la thérapie au patient ou lui rappeler la thérapie, sur la base de données déterminées, en particulier de données de mesure et de valeurs sauvegardées concernant la SpO2, le pouls, le CO2 et/ou un comportement changeant de la pression et/ou du débit volumique, lorsque les données déterminées indiquent qu'une élimination de sécrétions hors des voies respiratoires par une thérapie serait favorable.

5. Système selon l'une des revendications précédentes,
dans lequel le système est conçu pour modifier des paramètres de ventilation sur l'appareil respiratoire en fonction d'un effet déterminé de thérapies mises en œuvre sur une évolution de l'une au moins des grandeurs suivantes : la SpO2, le CO2, le flux, le volume tidal, le rapport signal-bruit (SNR).

6. Système selon l'une des revendications précédentes,
dans lequel l'appareil de thérapie et l'appareil respiratoire peuvent être accouplés pneumatiquement, de sorte qu'une ventilation et une insufflation et une exsufflation par machine peuvent être combinées directement.

7. Système selon l'une des revendications précédentes,
dans lequel le système est conçu de telle façon que le rappel de la thérapie ou le démarrage de la thérapie a lieu exclusivement ou également par le biais de l'appareil respiratoire accouplé.

8. Système selon l'une des revendications précédentes,
dans lequel les données, sur lesquelles se base l'au moins un mécanisme de thérapie, comportent une valeur de SpO2, dans lequel le système est conçu pour exécuter l'une au moins des fonctions suivantes, lorsque la valeur de SpO2 atteint une valeur seuil de SpO2 : rappel de la thérapie, démarrage de la thérapie, répétition de la thérapie, prolongement de la thérapie.

9. Système selon l'une des revendications précédentes,
dans lequel le système est conçu pour sauvegarder et/ou traiter un effet de la thérapie mise en œuvre sur une évolution de l'une au moins des grandeurs suivantes : la SpO2, le CO2, la pression, le flux, le volume tidal, des signaux acoustiques, un rapport signal-bruit (SNR) dans l'appareil de thérapie et/ou dans un appareil respiratoire accouplé ;
dans lequel le système est en outre conçu pour tenir compte des données sauvegardées et/ou traitées lors de décisions concernant l'une au moins des fonctions suivantes : démarrage de la thérapie, répétition de la thérapie, rappel de la thérapie.

10. Système selon l'une des revendications précédentes,
dans lequel le système est conçu pour évaluer le rapport signal-bruit (SNR) de signaux de pression et/ou de flux et pour initier ou prolonger un démarrage de la thérapie ou un rappel de la thérapie lorsque la valeur SNR atteint une valeur seuil de SNR.

11. Système selon l'une des revendications précédentes,
dans lequel le système est conçu pour effectuer automatiquement et/ou proposer au patient des répétitions d'insufflations et d'exsufflations, sur la base de données déterminées, en particulier de données de mesure et de valeurs sauvegardées concernant la pression, le flux, le volume tidal, la SpO2, le pouls et/ou le CO2, ou sur la base de données issues d'exercices pour la toux antérieurs, en particulier d'un flux de la toux, lorsque ces données indiquent que des sécrétions supplémentaires devraient être éliminées des voies respiratoires.

12. Système selon l'une des revendications précédentes,
dans lequel le système est conçu pour télécharger des données déterminées et/ou sauvegardées vers un système basé sur le cloud, prendre des décisions relatives à la thérapie au moyen d'algorithmes basés sur le cloud et mettre les décisions à la disposition de l'appareil de thérapie à partir du cloud pour l'adaptation de la thérapie ou de moments de démarrage de la thérapie.

13. Système selon l'une des revendications précédentes,
dans lequel le système est conçu pour démarrer et/ou répéter et/ou terminer un traitement de la toux côté utilisateur à la suite du rappel de la thérapie par l'appareil de thérapie, par le biais d'une télécommande et/ou d'une application sur un appareil terminal mobile couplé à l'appareil de thérapie.

14. Système selon l'une des revendications précédentes,
dans lequel le système est conçu pour démarrer automatiquement la thérapie sur la base de données déterminées, dans lequel les paramètres de thérapie sont adaptés automatiquement à l'aide des données déterminées.

15. Système selon l'une des revendications précédentes,
dans lequel l'au moins une unité de commande, l'au moins une mémoire, l'au moins une interface d'utilisateur et l'au moins un capteur font partie de l'appareil de thérapie.
